Europäisches Patentamt

European Patent Office

Office européen des brevets

(11) Veröffentlichungsnummer: **0 327 877**

**A2**

(12) **EUROPÄISCHE PATENTANMELDUNG**

(21) Anmeldenummer: 89101174.4

(22) Anmeldetag: 24.01.89

(51) Int. Cl.⁴: **C07K 5/02 , A61K 37/64**

Die Bezeichnung der Erfindung wurde geändert (Richtlinien für die Prüfung im EPA, A-III, 7.3).

(30) Priorität: 06.02.88 DE 3803584
07.06.88 DE 3819263

(43) Veröffentlichungstag der Anmeldung:
16.08.89 Patentblatt 89/33

(84) Benannte Vertragsstaaten:
AT BE CH DE ES FR GB IT LI NL SE

(71) Anmelder: MERCK PATENT GESELLSCHAFT MIT BESCHRÄNKTER HAFTUNG
Frankfurter Strasse 250 Postfach 4119
D-6100 Darmstadt(DE)

(72) Erfinder: Jonczyk, Alfred, Dr.
Scheppallee 57
D-6100 Darmstadt(DE)
Erfinder: Raddatz, Peter, Dr.
Grafenstrasse 37
D-6100 Darmstadt(DE)
Erfinder: Hölzemann, Günter, Dr.
Weedring 5
D-6104 Seeheim 1(DE)
Erfinder: Sombroek, Johannes, Dr.
Robert-Koch-Strasse 32
D-6100 Darmstadt 13(DE)
Erfinder: Schmitges, Claus J., Dr.
Karolinger Strasse 5
D-6114 Gross-Umstadt(DE)
Erfinder: Minck, Klaus-Otto, Dr.
Büchestrasse 8
D-6105 Ober-Ramstadt(DE)
Erfinder: Gante, Joachim, Dr.
Stormstrasse 4
D-6100 Darmstadt 12(DE)

(54) **Peptid-Renininhibitoren.**

(57) Neue Aminosäurederivate der Formel I

$$R^1\text{-}Z\text{-}M\text{-}NR^2\text{-}CHR^3\text{-}CR^4\text{-}(CHR^5)_n\text{-}CO\text{-}E\text{-}Q\text{-}Y \; An^{\ominus} \qquad I$$

worin
$R^1$, Z, M, $R^2$, $R^3$, $R^4$, $R^5$, n, E, Q, Y und $An^{\ominus}$ die in Patentanspruch 1 angegebene Bedeutung haben, sowie ihre Salze hemmen die Aktivität des menschlichen Plasmarenins.

## Aminosäurederivate

Die Erfindung betrifft neue Aminosäurederivate der Formel I

$R^1$-Z-M-$NR^2$-$CHR^3$-$CR^4$-$(CHR^5)_n$-CO-E-Q-Y $An^\ominus$     I

worin

$R^1$     $R^6$, $R^6$-O-$C_mH_{2m}$-CO-, $R^6$-$C_mH_{2m}$-O-CO-, $R^6$-$C_mH_{2m}$-CO-, $R^6$-$SO_2$-, ($R^6$-$C_mH_{2m}$-$(T)_x$-$(G)_y$-$C_rH_{2r}$)-L($R^7$-$C_pH_{2p}$)-$C_tH_{2t}$-CO-, $R^6$-$(NHCH_2CH_2)_m$-$NHCH_2CO$-, oder 9-Fluorenyl-$C_mH_{2m}$-O-CO-,

Z     0 bis 3 peptidartig miteinander verbundene Aminosäurereste ausgewählt aus der Gruppe bestehend aus Abu, Ada, Ala, βAla, Arg, Asn, Asp, Bia, Cal, Dab, Gln, Glu, Gly, His, N(im)-A-His, Ile, Leu, tert.-Leu, Lys, Met, αNal, βNal, Nbg, Nle, Orn, Phe, Pia, Pro, Pya, Ser, Thr, Tic, Trp, Tyr und Val,

M     βAla, Gly, Pia oder Pya, oder, falls im Rest Y anstelle eines N-Atoms einer Het-ylengruppe eine $R^8N^\oplus$-Gruppe steht, auch His,

E     0 bis 2 peptidartig miteinander verbundene Aminosäurereste ausgewählt aus der Gruppe bestehend aus Abu, Ala, Cal, His, Ile, Leu, Met, Nle, Nva, Phe, Trp, Tyr und Val,

Q     O oder NH,

Y     eine Alkylenkette, worin auch eine $CH_2$-Gruppe durch eine Ar-ylengruppe oder Het-ylengruppe ersetzt sein kann, mit insgesamt 1-20 C-Atomen, welche durch eine $R^8R^9R^{10}N^\oplus$-Gruppe oder eine unsubstituierte oder eine ein- oder mehrfach durch A, AO und/oder Hal substituierte Pyridiniumgruppe substituiert ist, und/oder in welcher an Stelle eines N-Atoms einer Het-ylengruppe eine $R^8N^\oplus$-Gruppe steht,

$An^\ominus$     ein Anion, das auch fehlen kann, wenn stattdessen eine in der Verbindung der Formel I enthaltene Carboxygruppe in Form eines Carboxylations vorliegt,

$R^3$, $R^6$ und $R^7$     jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^2$ und $R^5$     jeweils H oder A,

$R^4$     (H, OH), (H, $NH_2$) oder =O,

$R^8$, $R^9$ und $R^{10}$     jeweils Alkyl mit 1-18 C-Atomen oder Ar-alkyl, zwei der Reste $R^8$, $R^9$ und $R^{10}$ zusammen auch eine Alkylengruppe mit 2-8 C-Atomen, die durch ein O-Atom oder durch eine $NR^{11}$-Gruppe unterbrochen sein kann,

$R^{11}$     H, A, Ar oder Ar-alkyl,

L     CH oder N,

T     O, S, NH oder NA,

G     CO, S, SO oder $SO_2$,

n     1 oder 2,

m, p, r und t     jeweils 0, 1, 2, 3, 4 oder 5,

x und y     jeweils 0 oder 1,

Ar     unsubstituiertes oder ein- oder mehrfach durch A, AO, Hal, $CF_3$, OH, $H_2NSO_2$ und/oder $NH_2$ substituiertes Phenyl oder unsubstituiertes Naphthyl,

Het     einen gesättigten oder ungesättigten 5- oder 6-gliedrigen heterocyclischen Rest mit 1-4 N-, O- und/oder S-Atomen, der mit einem Benzolring kondensiert und/oder ein- oder mehrfach durch A, AO, Hal, $CF_3$ HO, $O_2N$, Carbonylsauerstoff, $H_2N$, HAN, $A_2N$, AcNH, AS, ASO, $ASO_2$, HOOC, AOOC, CN, $H_2NCO$, $H_2NSO_2$, $ASO_2NH$, Ar, Ar-alkyl, Ar-alkenyl, Hydroxyalkyl und/oder Aminoalkyl mit jeweils 1-8 C-Atomen substituiert sein kann und/oder dessen N- und/oder S-Heteroatome auch oxydiert sein können,

Hal     F, Cl, Br oder J,

Ac     A-CO-, Ar-CO- oder A-NH-CO-,

-alkyl-     eine Alkylengruppe mit 1-4 C-Atomen und

A     Alkyl mit 1-8 C-Atomen bedeuten,

worin ferner an Stelle einer oder mehrerer -NH-CO-Gruppen auch eine oder mehrere NA-CO-Gruppen stehen können,

sowie deren Salze.

Ähnliche Verbindungen sind aus der EP-A-163237, der EP-A-173481 und der EP-A-209897 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasmarenins. Diese Wirkung kann z.B. nach der Methode von F. Fyhrquist et al., Clin.Chem. 22, 250-256 (1976), nachgewiesen werden. Bemer-

2

kenswert ist, daß diese Verbindun gen sehr spezifische Hemmer des Renins sind; für die Hemmung anderer Aspartylproteinasen (z.B. Pepsin und Kathepsin D) sind in der Regel wesentlich höhere Konzentrationen dieser Verbindungen notwendig.

Die Verbindungen können als Arzneimittelwirkstoffe in der Human- und Veterinärmedizin eingesetzt werden, insbesondere zur Prophylaxe und zur Behandlung von Herz-, Kreislauf- und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen.

Die vor- und nachstehend aufgeführten Abkürzungen von Aminosäureresten stehen für die Reste -NR'-R"-CO-, in der Regel -NH-CHR-CO- (worin R, R' und R" die für jede Aminosäure bekannte spezifische Bedeutung haben) folgender Aminosäuren:

| | |
|---|---|
| Abu | 2-Aminobuttersäure |
| Ada | 3-(1-Adamantyl)-alanin |
| Ala | Alanin |
| ßAla | ß-Alanin |
| Arg | Arginin |
| Asn | Asparagin |
| Asp | Asparaginsäure |
| Bia | 3-(2-Benzimidazolyl)-alanin |
| Cal | 3-Cyclohexylalanin |
| Dab | 2,4-Diaminobuttersäure |
| Gln | Glutamin |
| Glu | Glutaminsäure |

| | |
|---|---|
| Gly | Glycin |
| His | Histidin |
| N(im)-A-His | in 1- oder 3-Stellung des Imidazolrings durch A substituiertes Histidin |
| Ile | Isoleucin |
| Leu | Leucin |
| tert.-Leu | tert-Leucin |
| Lys | Lysin |
| Met | Methionin |
| αNal | 3-(α-Naphthyl)-alanin |
| ßNal | 3-(ß-Naphthyl)-alanin |
| Nbg | (2-Norbornyl)-glycin |
| Nle | Norleucin |
| N-Me-His | N-Methyl-histidin |
| N-Me-Phe | N-Methyl-phenylalanin |
| Nva | Norvalin |
| Orn | Ornithin |
| Phe | Phenylalanin |
| Pia | 3-(Piperidyl)-alanin [z.B. 2-Pia 3-(2-Piperidyl)-alanin] |
| Pro | Prolin |
| Pya | 3-(Pyridyl)-alanin [z.B. 3-Pya 3-(3-Pyridyl)-alanin] |
| Ser | Serin |
| Thr | Threonin |
| Tic | 1,2,3,4-Tetrahydroisochinolin-1-carbonsäure |
| Trp | Tryptophan |
| Tyr | Tyrosin |
| Val | Valin. |

Ferner bedeutet nachstehend:

4

| ADPA | 2-Amino-5,6-dimethyl-3-pyrazinyl-methylamino |
|------|------|
| AMPA | 4-Amino-2-methyl-5-pyrimidinyl-methylamino |
| BOC | tert.-Butoxycarbonyl |
| imi-BOM | Benzyloxymethyl in 1-Stellung des Imidazolrings |
| CBZ | Benzyloxycarbonyl |
| DNP | 2,4-Dinitrophenyl |
| imi-DNP | 2,4-Dinitrophenyl in 1-Stellung des Imidazolrings |
| ETNC | N-Ethylcarbamoyl |
| ETOC | Ethoxycarbonyl |
| FMOC | 9-Fluorenylmethoxycarbonyl |
| IPNC | N-Isopropylcarbamoyl |
| IPOC | Isopropoxycarbonyl |
| MAC | Morpholinoacetyl |
| MC | Morpholinocarbonyl |
| OMe | Methylester |
| OEt | Ethylester |
| POA | Phenoxyacetyl |
| DCCI | Dicyclohexylcarbodiimid |
| HOBt | 1-Hydroxybenzotriazol. |

Sofern die vorstehend genannten Aminosäuren in mehreren enantiomeren Formen auftreten können, so sind vor- und nachstehend, z.B. als Bestandteil der Verbindungen der Formel I, alle diese Formen und auch ihre Gemische (z.B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Gegenstand der Erfindung ist ferner ein Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

$R^1$-$G^1$-OH     II

worin $G^1$

    (a) $Z^1$,

    (b) Z,

    (c) Z-M,

    (d) Z-M-W,

    (e) Z-M-W-$E^1$,

    (f) Z-M-W-E und

W     -$NR^2$-$CHR^3$-$CR^4$-$(CHR^5)_n$-CO- bedeuten

mit einer Aminoverbindung der Formel III

H-$G^2$     III

worin

$G^2$

    (a) -$Z^2$-M-W-E-Q-Y,

    (b) -M-W-E-Q-Y,

    (c) -W-E-Q-Y,

    (d) -E-Q-Y,

    (e) -$E^2$-Q-Y,

    (f) -NH-Y,

$E^1$ + $E^2$     zusammen E und

$Z^1$ + $Z^2$     zusammen Z bedeuten

umsetzt,

oder daß man ein sonst der Formel I entsprechendes tertiäres Amin mit einem quaternisierenden Mittel behandelt,

und daß man gegebenenfalls in einer Verbindung der Formel I ein Funktionell abgewandelte Amino- und/oder Hydroxygruppe durch Behandeln mit solvolysierenden oder hydrogenolysierenden Mitteln in Freiheit setzt und/oder zur Herstellung einer Verbindung der Formel I, $R^4$ = (H, OH) oder (H, NH$_2$) ein Aminoketosäurederivat der Formel I, $R^4$ = O, reduziert oder reduktiv aminiert und/oder eine Verbindung der Formel I durch Behandeln mit einer Säure in eines ihrer Salze überführt und/oder in einer Verbindung der Formel I ein Anion An$^\ominus$ gegen ein anderes Anion An$^\ominus$ austauscht.

Vor- und nachstehend haben die Reste bzw. Parameter $R^1$ bis $R^{11}$, Z, M, E, Q, Y, An$^\ominus$, L, T, G, m, n, p, r, t, x, y, Ar, Het, Hal, Ac, A, $G^1$, $G^2$, $E^1$, $E^2$, $Z^1$, $Z^2$ und W die bei den Formeln I, II oder III angegebenen Bedeutungen, falls nicht ausdrüklich etwas anderes angegeben ist.

In den vorstehenden Formeln hat A 1-8, vorzugsweise 1, 2, 3 oder 4 C-Atome. A bedeutet vorzugsweise Methyl, weiterhin, Ethyl, Propyl, Isopropyl, Butyl, Isobutyl, sek.-Butyl, oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, Heptyl, Octyl.

Cycloalkyl bedeutet vorzugsweise Cyclopropyl, Cyclobutyl, Cyclopentyl, Cyclohexyl oder Cycloheptyl, aber auch z.B. 1-, 2- oder 3-Methylcyclopentyl, 1-, 2-, 3- oder 4-Methylcyclohexyl.

Dementsprechend bedeutet Cycloalkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z.B. 1-, 2- oder 3-Methylcyclopentylmethyl, 1-, 2-, 3- oder 4-Methylcyclohexylmethyl.

Bicycloalkyl bedeutet vorzugsweise 1- oder 2-Dekalyl, 2-Bicyclo[2,2,1]heptyl oder 6,6-Dimethyl-2-bicyclo[3,1,1]heptyl.

Tricycloalkyl bedeutet vorzugsweise 1-Adamantyl.

Hal bedeutet vorzugsweise F, Cl oder Br, aber auch J.

Ac bedeutet vorzugsweise A-CO-, wie Acetyl, Propionyl oder Butyryl, Ar-CO- wie Benzoyl, o-, m- oder p-Methoxybenzoyl oder 3,4-Dimethoxybenzoyl, A-NH-CO- wie N-Methyl- oder N-Ethylcarbamoyl.

Ar bedeutet vorzugsweise Phenyl, ferner bevorzugt o-, m- oder p-Tolyl, o-, m- oder p-Ethylphenyl, o-, m- oder p-Methoxyphenyl, o-, m- oder p-Fluorphenyl, o-, m- oder p-Chlorphenyl, o-, m- oder p-Bromphenyl, o-, m- oder p-Jodphenyl, o-, m- oder p-Trifluormethylphenyl, o-, m-oder p-Hydroxyphenyl, o-, m- oder p-Sulfamoylphenyl, 2,3-, 2,4-, 2,5-, 2,6-, 3,4- oder 3,5-Dimethoxyphenyl, 3,4,5-Trimethoxyphenyl, o-, m- oder p-Aminophenyl, 1-oder 2-Naphthyl.

Dementsprechend bedeutet Ar-alkyl vorzugsweise Benzyl, 1- oder 2-Phenylethyl, o-, m- oder p-Methylbenzyl, 1-oder 2-o-, -m- oder -p-Tolylethyl, o-, m- oder p-Ethylbenzyl, 1- oder 2-o-, -m- oder -p-Ethylphenylethyl, o-, m- oder p-Methoxybenzyl, 1- oder 2-o-, -m- oder -p-Methoxyphenylethyl, o-, m- oder p-Fluorbenzyl, 1- oder 2-o-, -m- oder -p-Fluorphenylethyl, o-, m- oder p-Chlorbenzyl, 1- oder 2-o-, -m- oder -p-Chlorphenylethyl, o-, m- oder p-Brombenzyl, 1- oder 2-o-, -m- oder -p-Bromphenylethyl, o-, m- oder p-Jodbenzyl, 1- oder 2-o-, -m-oder -p-Jodphenylethyl, o-, m- oder p-Trifluormethylbenzyl, o-, m- oder p-Hydroxybenzyl, 2,3-, 2,4-, 2,5-, 2,6-, 3,4- oder 3,5-Dimethoxybenzyl, 3,4,5-Trimethoxybenzyl, o-, m- oder p-Aminobenzyl, 1- oder 2-Naphthylmethyl.

Het ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4- oder 5-Oxazolyl, 3-, 4-oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4-oder -5-yl, 1,2,4-Triazol-1-, -3- oder -5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 2,1,5-Thiadiazol-3- oder -4-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6-oder 7-Benzisoxazolyl, 2-, 4-, 5-, 6- oder 7-Benzthiazolyl, 2-, 4-, 5-, 6- oder 7-Benzisothiazolyl, 4-, 5-, 6-oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolyl, 2-, 4-, 5-, 6- 7- oder 8-Chinazolyl. Die heterocyclischen Reste können auch teilweise oder vollständig hydriert sein. Het kann also z.B. auch bedeuten 2,3-Dihydro-2-, -3-, -4- oder -5-furyl, 2,5-Dihydro-2-, -3-, -4- oder -5-furyl, Tetrahydro-2- oder -3-furyl, Tetrahydro-2- oder -3-thienyl, 2,3-Dihydro-1-, -2-, -3-, -4- oder -5-pyrryl, 2,5-Dihydro-1-, -2-, -3-, -4- oder -5-pyrryl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3-oder -4-pyridyl, 1,2,3,4-Tetrahydro-1-, -2-, -3-, -4-, -5-oder -6-pyridyl, 1,2,3,6-Tetrahydro-1-, -2-, -3-, -4-, -5-oder -6-pyridyl, 1-, 2-, 3- oder 4-Piperidinyl, 2-, 3-oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder 8-isochinolyl.

Die heterocyclischen Reste können auch wie angegeben substituiert sein. Het kann z.B. bevorzugt auch bedeuten: 2-Amino-4-thiazolyl, 4-Carboxy-2-thiazolyl, 4-Carbamoyl-2-thiazolyl, 4-(2-Aminoethyl)-2-thiazolyl,

6

2-Amino-5,6-dimethyl-3-pyrazinyl, 4-Carbamoylpiperidino, ferner z.B. 3-, 4- oder 5-Methyl-2-furyl, 2-, 4- oder 5-Methyl-3-furyl, 2,4-Dimethyl-3-furyl, 5-Nitro-2-furyl, 5-Styryl-2-furyl, 3-, 4- oder 5-Methyl-2-thienyl, 2-, 4- oder 5-Methyl-3-thienyl, 3-Methyl-5-tert.-butyl-2-thienyl, 5-Chlor-2-thienyl, 5-Phenyl-2- oder -3-thienyl, 1-, 3-, 4- oder 5-Methyl-2-pyrrolyl, 1-Methyl-4- oder -5-nitro-2-pyrrolyl, 3,5-Dimethyl-4-ethyl-2-pyrrolyl, 4-Methyl-5-pyrazolyl, 5-Methyl-3-isoxazolyl, 3,4-Dimethyl-5-isoxazolyl, 4- oder 5-Methyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 3-, 4-, 5-oder 6-Chlor-2-pyridyl, 2-, 4-, 5- oder 6-Chlor-3-pyridyl, 2- oder 3-Chlor-4-pyridyl, 2,6-Dimethyl-3-oder -4-pyridyl, 2,6-Dichlor-3- oder -4-pyridyl, 2-Hydroxy-3-, -4-, -5- oder -6-pyridyl (= 1H-2-Pyridon-3-, -4-, -5- oder -6-yl), 2-Hydroxy-4,6-dimethyl-3-pyridyl, 5-Phenyl-1H-2-pyridon-3-yl, 5-p-Methoxyphenyl-1H-2-pyridon-3-yl, 2-Methyl-3-hydroxy-4-hydroxymethyl-5-pyridyl, 2-Hydroxy-4-amino-6-methyl-3-pyridyl, 3-N'-Methylureido-1H-4-pyridon-5-yl, 4-Methyl-2-pyrimidinyl, 4,6-Dimethyl-2-pyrimidinyl, 2-, 5- oder 6-Methyl-4-pyrimidinyl, 2,6-Dimethyl-4-pyrimidinyl, 2,6-Dihydroxy-4-pyrimidinyl, 5-Chlor-2-methyl-4-pyrimidinyl, 2-Methyl-4-amino-5-pyrimidinyl, 3-Methyl-2-benzofuryl, 2-Ethyl-3-benzofuryl, 7-Methyl-2-benzothienyl, 1-, 2-, 4-, 5-, 6- oder 7-Methyl-3-indolyl, 1-Methyl-5- oder -6-benzimidazolyl, 1-Ethyl-5- oder -6-benzimidazolyl, 3-, 4-, 5-, 6-, 7- oder 8-Hydroxy-2-chinolyl, 2-Oxopyrrolidino, 2-Oxo-piperidino, 2,5-Dioxopyrrolidino, 3-Benzyl-2,5-dioxopyrrolidino.

L ist vorzugsweise CH. T ist vorzugsweise O oder S. G ist vorzugsweise CO. Die Parameter m, p, r und t sind vorzugsweise jeweils 0, 1 oder 2; n ist vorzugsweise 1; x ist vorzugsweise 0; y ist vorzugsweise 0 oder 1.

$R^1$ bedeutet vorzugsweise H, POA, Alkoxycarbonyl wie ETOC, IPOC oder BOC; Ar-alkoxycarbonyl wie CBZ; Het-alkoxycarbonyl wie 2-(2-, 3- oder 4-Pyridyl)-ethoxycarbonyl; Alkanoyl mit vorzugsweise 2-7 C-Atomen wie Acetyl, Propionyl, Butyryl, Isobutyryl, Pivaloyl (Trimethylacetyl), Hexanoyl, 3,3-Dimethylbutyryl (tert.-Butylacetyl), Heptanoyl, 2-Propylvaleryl (Dipropylacetyl); Amino-alkanoyl wie Aminoacetyl (H-Gly), 3-Aminopropionyl (H-βAla), 4-Aminobutyryl, 6-Aminohexanoyl, 8-Aminooctanoyl; Dialkylamino-alkanoyl wie 4-Dimethylaminobutyryl, 4-Diethylaminobutyryl oder 5-Dimethylaminopentanoyl; Amido-alkanoyl, z.B. BOC-Gly, ETOC-Gly, IPOC-Gly, BOC-βAla, 4-BOC-aminobutyryl, 6-BOC-aminohexanoyl, 8-BOC-aminooctanoyl, N-Benzoyl-N-benzyl-aminoacetyl; Cycloalkylcarbonyl wie Cyclopentylcarbonyl oder Cyclohexylcarbonyl; Ar-CO wie Benzoyl; Ar-alkanoyl wie Phenylacetyl, 2-oder 3-Phenylpropionyl, 4-Phenylbutyryl, 2-Benzyl-butyryl, 2-Benzylhexanoyl, 2-Benzyl-5-methyl-hexanoyl, 2-Benzyl-heptanoyl, 2- oder 3-o-, -m- oder -p-Fluorphenylpropionyl, 2- oder 3-o-, -m- oder p-Chlorphenylpropionyl; Oxo-Ar-alkanoyl, insbesondere Gruppen der Formel $R^{12}$-CO-CH$_2$-CH(CH$_2$C$_6$H$_5$)-CO-, worin $R^{12}$ A, Cycloalkyl mit 3-7 C-Atomen oder Cycloalkylalkyl mit 4-11 C-Atomen, z.B. Butyl, Isobutyl, tert.-Butyl, Pentyl, Isopentyl, 2,2-Dimethylpropyl, Hexyl, 4-Methylpentyl, 3,3-Dimethylbutyl, Heptyl, 5-Methylhexyl, Cyclohexyl, Cyclohexylmethyl oder 2-Cyclohexyl-ethyl bedeutet, wie 2-Benzyl-4-oxo-5,5-dimethylhexanoyl, 2-Benzyl-4-oxo-6,6-dimethyl-heptanoyl, 2-Benzyl-4-oxo-6-methyl-heptanoyl, 2-Benzyl-4-oxo-6-cyclohexyl-hexanoyl; durch Aminogruppen, insbesondere Dialkylaminogruppen substituiertes Ar-alkanoyl, z.B. 2-Benzyl-4-dimethylaminobutyryl, 2-Benzyl-5-dimethylaminopentanoyl; Di-Ar-alkanoyl wie 2-Benzyl-3-phenylpropionyl, 2-Benzyl-4-phenylbutyryl, 2-(2-Phenylethyl)-4-phenylbutyryl, 2-(1-oder 2-Naphthylmethyl)-4-phenyl butyryl; Het-alkanoyl wie Pyrrolidino-acetyl, 4-Pyrrolidinobutyryl, Piperidinoacetyl, 4-Aminopiperidinoacetyl, MAC; Di-Het-alkanoyl wie 2-(2,5-Dioxopyrrolidino)-3-(3-indolyl)-propionyl, 2-(3-Benzyl-2,5-dioxopyrrolidino-3-(3-indolyl)-propionyl; Cycloalkylalkanoyl wie Cyclohexylacetyl, 2- oder 3-Cyclohexylpropionyl; FMOC; heterocyclisch substituiertes Arylalkanoyl wie 2-Benzyl-4-(2-, -3- oder -4-pyridyl)-butyryl, 2-Benzyl-4-(2-oxopyrrolidino)-butyryl, 2-(2,5-Dioxopyrrolidino)-3-phenylpropionyl, 2-(2,5-Dioxopyrrolidino)-3-p-hydroxyphenylpropionyl, 2-(2,5-Dioxopyrrolidino)-3-(1- oder -2-naphthyl)-propionyl, 2-(3-Benzyl-2,5-dioxopyrrolidino)-3-phenyl-propionyl, 2-(3-Benzyl-2,5-dioxopyrrolidino)-3-hydroxyphenyl-propionyl, 2-(3-Benzyl-2,5-dioxopyrrolidino)-3-(1- oder -2-naphthyl)-propionyl; N-substituiertes Carbamoyl wie ETNC, IPNC, N-Benzylcarbamoyl, N-(2-, 3- oder 4-Pyridylmethyl)-carbamoyl; N,N-disubstituiertes Carbamoyl wie N,N-Dibenzyl-carbamoyl, N-Benzyl-N-2-phenylethyl-carbamoyl, N-Benzyl-N-butyl-carbamoyl, N-Benzyl-N-isopentyl-carbamoyl, N,N-Bis-2-phenylethylcarbamoyl, N-2-Morpholinoethyl-N-1-naphthylmethylcarbamoyl, Pyrrolidinocarbonyl, Piperidinocarbonyl, 4-Aminopiperidinocarbonyl, MC; durch Alkoxycarbonyl substituiertes Arylalkanoyl wie 2-Benzyl-3-ethoxycarbonylpropionyl, 2-(1-Naphthylmethyl)-3-ethoxycarbonylpropionyl; durch substituiertes Carbamoyl substituiertes Arylalkanoyl wie 2-Benzyl-3-(N-isopropyl-carbamoyl)propionyl, 2-(1-Naphthylmethyl)-3-(N-isopropylcarbamoyl)-propionyl, 2-(1-Naphthylmethyl)-3-(N-2-phenylethylcarbamoyl)-propionyl, 2-Benzyl-3-piperidinocarbonyl-propionyl, 2-Benzyl-3-morpholinocarbonylpropionyl, 2-(1-Naphthylmethyl)-3-morpholinocarbonylpropionyl; durch Acyl substituiertes Arylalkanoyl wie 2-Benzyl-4-oxo-hexanoyl, 2-Benzyl-4-oxo-5,5-dimethylhexanoyl, 2-Benzyl-4-oxo-6-methyl-heptanoyl, 2-Benzyl-4-oxo-4-phenylbutyryl, 2-Benzyl-4-oxo-4-(2-, (3- oder (4-pyridyl)-butyryl; Alkylsulfonyl wie tert.-Butylsulfonyl; durch Alkylsulfonyl substituiertes Arylalkanoyl wie 2-tert.-Butylsulfonylmethyl-3-phenylpropionyl.

Einige besonders bevorzugte Reste $R^1$ sind BOC, ferner Pivaloyl, 3,3-Dimethylbutyryl, 4-Aminobutyryl,

7

6-Aminohexanoyl, 8-Aminooctanoyl, 4-Dimethylaminobutyryl, 5-Dimethylaminopentanoyl, 2-Benzyl-5-dimethylaminopentanoyl, MAC, 2-(2,5-Dioxopyrrolidino)-3-phenylpropionyl, 2-(3-Benzyl-2,5-dioxopyrrolidino)-3-phenylpropionyl, 4-Amino-piperidinocarbonyl, 4-Amino-piperidinoacetyl.

Z bedeutet vorzugsweise einen, aber auch 0 oder 2, weiterhin 3 peptidartig miteinander bzw. mit den Gruppen $R^1$ und $NR^2$ verbundene Aminosäurereste, insbesondere die Gruppe Phe, weiterhin eine der Gruppen αNal, βNal, Trp oder Tyr, ferner bevorzugt eine der Gruppen Abu, Ada, Asn, Bia, Cal, Gln, Gly, His, N(im)-Methyl-His, Leu, Nle, Pia, Pya, Abu-His, Ada-His, Ala-His, Ala-Phe, Asn-His, Bia-His, Cal-His, Dab-His, Glu-His, Gly-His, His-His, Ile-His, Leu-His, tert.-Leu-His, Lys-His, Met-His, αNal-His, βNal-His, Nbg-His, Nle-His (N-Me-His)-His, (N-Me-Phe)-His, Orn-His, Phe-Abu, Phe-Ada, Phe-Ala, Phe-Arg, Phe-Asn, Phe-Bia, Phe-Cal, Phe-Dab, Phe-Gln, Phe-Glu, Phe-Gly, Phe-(N-im-Methyl-His), Phe-Ile, Phe-Leu, Phe-tert.-Leu, Phe-Lys, Phe-Met, Phe-α-Nal, Phe-βNal, Phe-Nbg, Phe-Nle, Phe-(N-Me-His), Phe-(N-Me-Phe), Phe-Orn, Phe-Phe, Phe-Pro, Phe-Ser, Phe-Thr, Phe-Tic, Phe-Trp, Phe-Tyr, Phe-Val, Pro-His, Ser-His, Thr-His, Tic-His, Trp-His, Tyr-His, Val-His, ferner Ada-Phe-His, Pro-Ala-His, Pro-Ala-Phe, Pro-Phe-Ala, Pro-Phe-Phe, weiterhin Pro-Abu-His, Pro-Arg-His, Pro-Asn-His, Pro-Bia-His, Pro-Dab-His, Pro-Glu-His, Pro-His-His, Pro-Ile-His, Pro-Leu-His, Pro-tert.-Leu-His, Pro-Lys-His, Pro-Met-His, Pro-Nbg-His, Pro-Nle-His, Pro-(N-Me-His)-His, Pro-(N-Me-Phe)-His, Pro-Orn-His, Pro-Phe-Abu, Pro-Phe-Ada, Pro-Phe-Arg, Pro-Phe-Asn, Pro-Phe-Bia, Pro-Phe-Dab, Pro-Phe-Gln, Pro-Phe-Glu, Pro-Phe-His, Pro-Phe-(N-im-Methyl-His), Pro-Phe-Ile, Pro-Phe-Leu, Pro-Phe-tert.-Leu, Pro-Phe-Lys, Pro-Phe-Met, Pro-Phe-Nbg, Pro-Phe-Nle, Pro-Phe-(N-Me-His), Pro-Phe-(N-Me-Phe), Pro-Phe-Orn, Pro-Phe-Pro, Pro-Phe-Ser, Pro-Phe-Thr, Pro-Phe-Tic, Pro-Phe-Trp, Pro-Phe-Tyr, Pro-Phe-Val, Pro-Pro-His, Pro-Ser-His, Pro-Thr-His, Pro-Tic-His, Pro-Trp-His, Pro-Tyr-His, Pro-Val-His.

Falls $R^1$ eine der Gruppen $R^0$-CH(CH$_2$C$_6$H$_5$)-CO- bedeutet, worin $R^0$ = $R^6$-C$_m$H$_{2m}$-(T)$_x$-(G)$_y$-C$_r$H$_{2r}$ ist, vorzugsweise jedoch Pyrrolidino, 2-Oxo-pyrrolidino, 2,5-Dioxopyrrolidino, 3-Benzyl-2,5-dioxopyrrolidino, Piperidino, Morpholino, Alkyl, Alkoxy oder Alkylthio mit jeweils 1-8 C-Atomen oder A$_2$N-C$_r$H$_{2r}$ bedeutet, ist Z vorzugsweise abwesend.

M ist vorzugsweise Gly, ferner bevorzugt βAla oder 3-Pya.

E ist vorzugsweise abwesend oder bedeutet vorzugsweise Ile oder Leu, ferner bevorzugt Abu, Cal, Met, Nle, Phe oder Val.

Q ist vorzugsweise NH.

$R^2$ und $R^5$ bedeuten vorzugsweise H oder Methyl, ferner Ethyl, Propyl, Isopropyl, Butyl oder Isobutyl. Falls n = 2 ist, können die beiden Reste $R^5$ gleich oder voneinander verschieden sein; vorzugsweise ist in diesem Fall der eine Rest $R^5$ H, der andere A, insbesondere Isopropyl.

$R^3$ bedeutet vorzugsweise Cyclohexylmethyl, ferner bevorzugt A, insbesondere Methyl, Ethyl, Propyl, Isopropyl, Butyl, Isobutyl, sek.-Butyl, Pentyl, Isopentyl (3-Methylbutyl) oder 2-Methylbutyl, Phenyl, Benzyl, p-Chlorbenzyl, 2-Cyclohexylethyl, Bicyclo[2,2,1]heptyl-2-methyl oder 6,6-Dimethylbicyclo[3,1,1]heptyl-2-methyl.

$R^4$ ist vorzugsweise (H, OH).

$R^6$ und $R^7$ bedeuten vorzugsweise A, insbesondere Methyl, Ethyl, Propyl, Isopropyl, Butyl, Isobutyl oder tert.-Butyl, weiterhin vorzugsweise Cyclopropyl, Cyclopentyl, Cyclohexyl, Phenyl, Benzyl, Pyrrolidino, Piperidino oder Morpholino.

$R^8$, $R^9$, $R^{10}$ bedeuten vorzugsweise jeweils A; bevorzugt sind sie gleich und bedeuten jeweils Methyl oder Ethyl, einer dieser Reste bevorzugt auch höheres Alkyl mit vorzugsweise 6-18 C-Atomen oder Benzyl, zwei dieser Reste bevorzugt auch Tetramethylen, Pentamethylen, 3-Oxapentamethylen oder 3-NR$^{11}$-pentamethylen.

$R^{11}$ ist vorzugsweise H, A, Phenyl oder Benzyl.

Die Gruppe $R^8R^9R^{10}N^{\oplus}$, nachstehend kurz als "Am" bezeichnet, bedeutet dementsprechend vorzugsweise Trialkylammonium, insbesondere Trimethyl- oder Triethylammonium, ferner bevorzugt Dimethyl-butyl-, -pentyl-, -hexyl-, -heptyl-, -octyl-, -nonyl-, -decyl-, -undecyl-, -dodecyl-, -tetradecyl-, -hexadecyl- oder -octadecylammonium, N-Alkylpyrrolidinium, N-Alkyl-piperidinium, N-Alkylmorpholinium.

Die Gruppe Y ist bevorzugt -Alkylen-Am, insbesondere -CH$_2$CH$_2$-Am oder -CH$_2$CH$_2$CH$_2$-Am, ferner bevorzugt -CH$_2$-p-C$_6$H$_4$-CH$_2$-Am; -Alkylen-(1-Pyridinium), insbesondere -CH$_2$CH$_2$-(1-Pyridinium) oder -CH$_2$CH$_2$CH$_2$-(1-Pyridinium); -Alkylen-(2-, -(3 oder -(4-N-alkyl-pyridinium; N,N-Dialkyl-2-, -3- oder -4-piperidinium; N-Alkyl-N-Ar-alkyl-2-, -2- oder -4-piperidinium; Alkylen-2-, -3- oder -4-N,N-dialkyl-piperidinium; Alkylen-2-, -3- oder -4-N-alkyl-N-Ar-alkyl-piperidinium. Ferner ist die Gruppe Y bevorzugt 1- (oder 3-) A-4-amino-2-methyl-pyrimidinium-5-methyl, 1-A-2,6-dimethylpyridinium-3-methyl, 1-A-2-hydroxy-4,6-dimethyl-pyridinium-3-methyl, 3-A-thiazolium-4- oder -5-methyl, 3-A-benzthiazolium-2-methyl.

Die Gruppe W bedeutet vorzugsweise -NH-CHR$^3$-CHOH-CH$_2$-CO-, insbesondere -NH-CH-(Cyclohexylmethyl)-CHOH-CH$_2$-CO-("AHCP"; abgeleitet von 4-Amino-3-hydroxy-5-cyclohexylpentansäure),

ferner -NH-CH(CH$_2$CH$_2$-Cyclohexyl)-CHOH-CH$_2$-CO- ("AHCH"; abgeleitet von 4-Amino-3-hydroxy-6-cyclohexylhexansäure), -NH-CH(Isobutyl)-CHOH-CH$_2$-CO- ("Sta"; abgeleitet von Statin) oder -NH-CH(Benzyl)-CHOH-CH$_2$-CO ("AHPP"; abgeleitet von 4-Amino-3-hydroxy-5-phenylpentansäure). Die Gruppe W bedeutet ferner bevorzugt -NH-CHR$^3$-CH(NH$_2$)-CH$_2$-CO-, insbesondere -NH-CH(Cyclohexylmethyl)-CH(NH$_2$)-CH$_2$-CO- ("DACP"; abgeleitet von 3,4-Diamino-5-cyclohexylpentansäure), -NH-CH(CH$_2$CH$_2$-Cyclohexyl)-CH(NH$_2$)-CH$_2$-CO- ("DACH"; abgeleitet von 3,4-Diamino-6-cyclohexylhexansäure), -NH-CH(Isobutyl)-CH(NH$_2$)-CH$_2$-CO- ("DAMH"; abgeleitet von 3,4-Diamino-6-methylheptansäure) oder -NH-CH(Benzyl)-CH(NH$_2$)-CH$_2$-CO- ("DAPP"; abgeleitet von 3,4-Diamino-5-phenylpentansäure.

Die Gruppe W besitzt mindestens ein chirales Zentrum. In den Gruppen R$^1$, Z, M, E und Y können weitere chirale Zentren vorhanden sein. Die Verbindungen der Formel I können daher in verschiedenen - optisch-inaktiven oder optischaktiven - Formen vorkommen. Die Formel I umschließt alle diese Formen. Falls W -NH-CHR$^3$-CR$^4$-CH$_2$-CO- mit R$^4$ = (H, OH) oder (H, NH$_2$) bedeutet, sind die 3S-Hydroxy-4S-amino-Enantiomeren bzw. 3S,4S-Diamino-Enantiomeren bevorzugt. Wenn bei der Bezeichnung von Einzelsubstanzen nichts anderes angegeben ist, beziehen sich die Abkürzungen AHCP, AHCH, Sta, AHPP, DACP, DACH, DAMH und DAPP immer auf die 3S,4S-Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ih ausgedrückt werden, die der Formel I entsprechen, worin jedoch

in Ia

R$^1$      A-O-CO-, A-CO-, A$_2$N-C$_t$H$_{2t}$-CO- oder R$^0$-CH(CH$_2$CH$_6$H$_5$)-CO- bedeutet;

in Ib

R$^1$      BOC bedeutet;

in Ic

Z      Phe bedeutet;

in Id

M      βAla, Gly oder 3-Pya bedeutet;

in Ie

-NR$^2$-CHR$^3$-CR$^4$-(CHR$^5$)$_n$-CO- (= W) AHCP bedeutet;

in If

E      Ile oder Leu bedeutet;

In Ig

R$^1$-Z      A-O-CO-Phe, A-CO-Phe, -A-CO-CH$_2$-CH(CH$_2$-C$_6$H$_5$)-CO- oder 2,5-Dioxopyrrolidino-CH-(CH$_2$-C$_6$H$_5$)-CO-,

M      β-Ala, Gly oder 3-Pya,

W      AHCP oder 2-Isopropyl-3-hydroxy-4-amino-5-cyclohexyl-pentanoyl und

E      Ile oder Leu bedeuten;

in Ih

R$^1$-Z      BOC-Phe,

M      β-Ala, Gly oder 3-Pya,

W      AHCP und

E      Ile oder Leu bedeuten.

Insbesondere sind bevorzugt Verbindungen der Teilformeln:

I* sowie Ia* bis Ih*, die den Formeln I sowie Ia bis Ih entsprechen, worin jedoch

Q-Y      -NH-(CH$_2$)$_z$-Am, -NH-CH$_2$-p-C$_6$H$_4$-CH$_2$Am, -NH-(CH$_2$)$_z$-(1-pyridinium) oder -NH-(CH$_2$)$_z$-(1A-2-, -3- oder -4-pyridinium),

z      1, 2, 3, 4 oder 5 und

Am      Tris-C$_{1-18}$-alkyl-ammonium, 1-A-pyrrolidinium, 1-A-piperidinium oder 1-A-morpholinium bedeuten;

I' sowie Ia' bis Ih', die den Formeln I sowie Ia bis Ih entsprechen, worin jedoch

Q-Y      -NH-(CH$_2$)$_z$-NA$_3^{\oplus}$ und

z      2 oder 3 bedeuten;

I'' sowie Ia'' bis Ih'', die den Formeln I sowie Ia bis Ih entsprechen, worin jedoch

Q-Y      -NH-CH$_2$-(1- oder 3-A-4-amino-2-methyl-5-pyrimidinium), oder -NH-CH$_2$-(1-A-2-, 3- oder 4-pyridinium) bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; ferner EP-A-45665, EP-A-77028, EP-A-77029, EP-A-81783) beschrieben sind, und zwar unter Reaktionsbedingungen, die für die

9

genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Die Verbindungen der Formel I können erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber an Stelle einer oder mehrerer freier Amino- und/oder Hydroxygruppen entsprechende geschützte Amino- und/oder Hydroxygruppen enthalten, vorzugsweise solche, die an Stelle eines H-Atoms, das mit einem N-Atom verbunden ist, eine Aminoschutzgruppe tragen, z.B. solche die der Formel I entsprechen, aber an Stelle einer His-Gruppe eine N(im)-R'-His-Gruppe (worin R' eine Aminoschutzgruppe bedeutet, z.B. BOM oder DNP) enthalten, oder solche der Formel $R^1$-Z-M-$NR^2$-$CHR^3$-CH(NHR')-$(CHR^5)_n$-CO-E-Q-Y.

Ferner sind Ausgangsstoffe bevorzugt, die an Stelle des H-Atoms einer Hydroxygruppe eine Hydroxyschutzgruppe tragen, z.B. solche der Formel $R^1$-Z-M-$NR^2$-$CHR^3$-CHOR''-$(CHR^5)_n$-CO-E-Q-Y, worin R'' eine Hydroxyschutzgruppe bedeutet.

Es können auch mehrere - gleiche oder verschiedene -geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an einer anderen Stelle des Moleküls durchgeführt worden ist. Typisch für solche Gruppen sind insbesondere unsubstituierte oder substituierte Acyl-, Aryl- (z.B. DNP), Aralkoxymethyl- (z.B. BOM) oder Aralkylgruppen (z.B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren im weitesten Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, ETOC, 2,2,2-Trichlorethoxycarbonyl, BOC, 2-Jodethoxycarbonyl; Aralkyloxycarbonyl wie CBZ ("Carbobenzoxy"), 4-Methoxybenzyloxycarbonyl, FMOC. Bevorzugte Aminoschutzgruppen sind BOC, DNP und BOM, ferner CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an einer anderen Stelle des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butyl, Benzyl, p-Nitrobenzoyl, p-Toluolsulfonyl und Acetyl, wobei Benzyl und Acetyl besonders bevorzugt sind.

Die als Ausgangsstoffe zu verwendenden funktionellen Derivate der Verbindungen der Formel I können nach üblichen Methoden der Aminosäure- und Peptidsynthese hergestellt werden, wie sie z.B. in den genannten Standardwerken und Patentanmeldungen beschrieben sind, z.B. auch nach der Festphasenmethode nach Merrifield.

Das In-Freiheit-Setzen der Verbindungen der Formel I aus ihren funktionellen Derivaten gelingt - je nach der benutzten Schutzgruppe - z.B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol-oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluoressigsäure wird vorzugsweise im Überschuß ohne Zusatz eines weiteren Lösungsmittels verwendet, Perchlorsäure in Form eines Gemisches aus Essigsäure und 70%iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die BOC-Gruppe kann z.B. bevorzugt mit 40%iger Trifluoressigsäure in Methylenchlorid oder mit etwa 3 bis 5 n HCl in Dioxan bei 15-30° abgespalten weden, die FMOC-Gruppe mit einer etwa 5-20%igen Lösung von Dimethylamin, Diethylamin oder Piperidin in DMF bei 15-30°. Eine Abspaltung der DNP-Gruppe gelingt z.B. auch mit einer etwa 3-10%igen Lösung von 2-Mercaptoethanol und DMF/Wasser bei 15-30°.

Hydrogenolytisch entfernbare Schutzgruppen (z.B. BOM, CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z.B. gut an 5-10%igem Pd-C in Methanol bei 20-30°. Verbindungen der Formel I können auch durch direkte Peptidsynthese aus einer Carbonsäure- (Formel II) und einer Aminkomponente (Formel III) erhalten werden. Als Carbonsäurekomponenten eignen sich z.B. solche der Teilformeln $R^1$-Z-OH, $R^1$-Z-M-OH, $R^1$-Z-M-W-OH oder $R^1$-Z-M-W-E-OH, als Aminkomponenten solche der Teilformeln H-M-W-E-Q-Y, H-W-E-Q-Y, H-E-Q-Y oder $H_2$N-Y. Die Peptidbindung kann aber auch innerhalb der Gruppe Z bzw. E geknüpft werden; dabei wird eine Carbonsäure der Formel $R^1$-$Z^1$-OH bzw. $R^1$-Z-M-W-$E^1$-OH mit einer Aminoverbindung der Formel H-$Z^2$-M-W-E-Q-Y bzw. H-$E^2$-Q-Y umgesetzt, wobei $Z^1$ + $Z^2$ = Z bzw. $E^1$ + $E^2$ = E ist. Dabei arbeitet man zweckmäßig nach üblichen Methoden der Peptid-Synthese, wie sie z.B. in Houben-Weyl, 1.c., Band 15/II, Seiten 1-806 (1974) beschrieben sind.

Die Reaktion gelingt vorzugsweise in Gegenwart eines Dehydratisierungsmittels, z.B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z.B. solche, in denen reaktive Gruppen intermediär durch Schutzgruppen blockiert sind. Die Aminosäurederivate III können z.B. in Form ihrer aktivierten Ester verwendet werden, die zweckmäßig in situ gebildet werden, z.B. durch Zusatz von HOBt oder N-Hydroxysuccinimid.

Die Ausgangsstoffe der Formeln II und III sind großenteils bekannt. Sofern sie nicht bekannt sind, können sie nach bekannten Methoden, z.B. den oben angegebenen Methoden der Peptidsynthese und der Abspaltung von Schutzgruppen hergestellt werden.

Die Verbindungen der Formel I sind ferner erhältlich, indem man ein tertiäres Amin, das sonst der Formel I entspricht, mit einem quaternisierenden Mittel behandelt.

Als tertiäre Amine eignen sich z.B. (a) Verbindungen, die der Formel I entsprechen, aber an Stelle des Restes Y eine Alkylenkette enthalten, worin auch eine $CH_2$-Gruppe durch eine Ar-ylengruppe oder Het-ylengruppe ersetzt sein kann, mit insgesamt 1-20 C-Atomen, welche durch eine $R^9R^{10}$N-Gruppe oder eine unsubstituierte oder eine ein- oder mehrfach durch A, AO und/oder Hal substituierte Pyridylgruppe substituiert ist. Ferner eignen sich (b) Pyridin oder Amine der Formel $R^8R^9R^{10}$N. Als quaternisierende Mittel eignen sich im Fall (a) Halogenide der Formel $R^8$X (X = Cl, Br oder J), z.B. Alkylhalogenide wie Methyljodid oder Ethylbromid, oder Ar-alkylhalogenide wie Benzylchlorid, im Fall (b) dagegen z.B. Halogenide der Formel $R^1$-Z-M-W-E-Q-alkylen-X, z.B. BOC-Phe-Gly-AHCP-Ile-N-(2-bromethyl)-amid oder 2-(2,5-Dioxopyrrolidino)-3-phenylpropionyl-Gly-AHCP-Ile-N-(3-chlorpropyl)-amid.

Man quaternisiert zweckmäßig in Gegenwart eines der oben angegebenen inerten Lösungsmittel, z.B. Acetonitril, bei Temperaturen zwischen etwa -10 und +40°.

Gewünschtenfalls kann in einer Verbindung der Formel I eine funktionell abgewandelte Amino- und/oder Hydroxygruppe durch Solvolyse oder Hydrogenolyse nach einer der oben beschriebenen Methoden in Freiheit gesetzt werden.

So kann insbesondere eine Verbindung der Formel I, worin $R^1$ von H umgewandelt verschieden ist, in eine Verbindung der Formel I ($R^1$ = H) werden, zweckmäßig durch Hydrogenolyse, falls $R^1$ = CBZ ist, sonst durch selektive Solvolyse. Falls $R^1$ = BOC ist, kann man z.B. mit HCl in Dioxan bei Raumtemperatur die BOC-Gruppe abspalten.

Weiterhin können z.B. Ketoverbindungen der Formel I ($R^4$ = O) zu Verbindungen der Formel I ($R^4$ = (H, OH)) reduziert werden, beispielsweise mit einem komplexen Metallhydrid wie $NaBH_4$, das nicht

gleichzeitig die Peptid-Carbonylgruppen reduziert, in einem inerten Lösungsmittel wie Methanol bei Temperaturen zwischen etwa -10 und +30°.

Ketoverbindungen der Formel I ($R^4$ = O) können auch durch reduktive Aminierung in Verbindungen der Formel I ($R^4$ = H, $NH_2$) übergeführt werden. Man kann ein- oder mehrstufig reduktiv aminieren. So kann man z.B. die Ketoverbindung mit Ammoniumsalzen, z B. Ammoniumacetat, und $NaCNBH_3$ behandeln, vorzugsweise in einem inerten Lösungsmittel, z.B. einem Alkohol wie Methanol, bei Temperaturen zwischen etwa 0 und 50°, insbesondere zwischen 15 und 30°. Weiterhin ist es möglich, die Ketoverbindung zunächst mit Hydroxylamin in üblicher Weise in das Oxim zu überführen und dieses, z.B. durch katalytische Hydrierung an Raney-Nickel, zum Amin zu reduzieren.

Eine Base der Formel I kann mit einer Säure in das zugehörige Säureadditionssalz übergeführt wrden. Für diese Umsetzung kommen insbesondere Säuren in Frage die physiologisch unbedenkliche Salze liefern, So können anorganische Säuren verwendet werden, z.B. Schwefelsäure Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren z.B. Ameisensäure, Essigsäure, Propionsäure, Pivalinsäure Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure Nicotinsäure, Isonicotinsäure, Methan- oder Ethansulfonsäure, Ethandisulfonsäure, 2-Hydroxyethansulfonsäure, Benzolsulfonsäure p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Es ist ferner möglich, in einer Verbindung der Formel I ein Anion $An^{\ominus}$ gegen ein anderes Anion $An^{\ominus}$ auszutauschen. Als Anionen $An^{\ominus}$ eignen sich die Anionen der vorstehend genannten Säuren; bevorzugt sind die Chloride, Bromide Methansulfonate, Acetate, Citrate, Fumarate, Maleate und Succinate. Zum Anionenaustausch kann man z.B. den Ausgangsstoff einer Gelchromatographie unterziehen, wobei man als Eluens ein Lösungsmittelgemisch verwendet, das das gewünschte Anion im Überschuß enthält. Als Träger eignet sich vorzugsweise ein vernetztes Polydextran. Will man ein Acetat erhalten, so arbeitet man z.B. mit Essigsäure/Wasser oder Methanol/Essigsäure/Wasser als Eluens.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff-(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate, Für die Applikation als Inhalations-Spray können Sprays verwendet werden, die den Wirkstoff entweder gelöst oder suspendiert in einem Treibgasgemisch (z.B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z.B. Ethanol. Inhalationslösungen können mit Hilfe üblicher Inhalatoren verabfolgt werden. Die neuen Verbindungen können auch lyophilisiert und die erhaltenen Lyophilisate z.B. zur Hersetllung von Injek tionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-163227, der EP-A-173481 und der EP-A-209897 beschriebenen Verbindungen verabreicht, vorzugsweise in Dosierungen zwischen etwa 100 mg und 30 g, insbesondere zwischen 500 mg und 5 g pro Dosierungseinheit. Die tägliche Dosierung liegt vorzugsweise zwischen etwa 2 und 600 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt je nach Konstitution des Endprodukts auf pH-Werte zwischen 2 und 8 ein, extrahiert mit Ethylacetat oder Dichlormethan, trennt ab, trocknet die organische Phase über Natriumsulfat, filtriert, dampft ein, unterzieht das in der Regel ölige Rohprodukt einer Gelfiltration an vernetztem Polydextran mit Methanol/Wasser oder Essigsäure/Wasser als Eluens, dampft erneut ein und reinigt durch Chromatographie an Kieselgel und/oder Kristallisation. Wenn bei der Gelfiltration das Eluens Essigsäure enthält, erhält man die quartären Acetate der Formel I, An = $CH_3COO$. Die Rf-Werte sind dünnschichtchromatographisch an Kieselgel erhalten; Eluens Methanol/Essigsäure/Wasser 7:1:2, wenn nicht anders angegeben.

Beispiel 1

Ein Gemisch von 1,079 g N-2-[3S-Hydroxy-4S-(N-tert.-butoxycarbonyl-L-phenylalanyl-N(imi)-(2,4-dinitrophenyl)-L-histidyl-glycyl-amino)-5-cyclohexyl-pentanoyl-L-isoleucyl-amino]-ethyl-N,N,N-trimethyl-ammoniumacetat ["N-2-(BOC-Phe-(imi-DNP-His)-Gly-AHCP-Ile-amino)-ethyl-N,N,N-trimethylammoniumacetat"; erhältlich durch Reaktion von BOC-Ile-N-2-dimethylaminoethyl-amid (F. 100-105°) mit $CH_3I$ zu N-2-(BOC-Ile-amino)-ethyl-N,N,N-trimethylammoniumiodid (Öl; Rf 0,45, Trichlorethan/Methanol/Essigsäure 6:3:1), Abspaltung der BOC-Gruppe mit HCl/Dioxan zu N-2-(H-Ile-amino)-ethyl-N,N,N-trimethylammoniumchlorid-hydrochlorid (F. 89-120°) und Kondensation mit BOC-Phe-(imi-DNP-His)-Gly-AHCP-OH], 2 g 2-Mercaptoethanol, 20 ml DMF und 20 ml Wasser wird unter Rühren bei 20° mit wässeriger $Na_2CO_3$-Lösung auf pH 8 eingestellt und 2 Std. bei 20° gerührt. Nach üblicher Aufarbeitung erhält man N-2-[3S-Hydroxy-4S-(N-tert.-butoxycarbonyl-L-phenylalanyl-L-histidyl-glycyl-amino)-5-cyclohexyl-pentanoyl-L-isoleucyl-amino]-ethyl-N,N,N-trimethylammoniumacetat ["N-2-(BOC-Phe-His-Gly-AHCP-Ile-amino)-ethyl-N,N,N-trimethylammoniumacetat"].

Beispiel 2

Man löst 1 g N-2-[BOC-Phe-(CBZ-3-Pia)-AHCP-Ile-amino]-ethyl-N,N,N-trimethylammoniumacetat [erhältlich durch Hydrierung von BOC-Phe-3-Pya-AHCP-OH zu BOC-3-Pia-AHCP-OH, Reaktion mit CBZ-Cl zu BOC-(CBZ-3-Pia)-AHCP-OH, Kondensation mit N-(2-H-Ile-aminoethyl)-N,N,N-trimethylammoniumchlorid-hydrochlorid und HPLC mit Ammoniumacetat] in 15 ml Ethanol, hydriert 3 Std. an 0,5 g 10%ig. Pd-C bei 20° und 1 bar, filtriert, dampft ein und erhält nach chromatographischer Reinigung N-2-(BOC-Phe-3-Pia-AHCP-Ile-amino)-ethyl-N,N,N-trimethylammoniumacetat.

Analog erhält man durch Hydrogenolyse der entsprechenden CBZ-Derivate:

N-2-(BOC-Phe-2-Pia-AHCP-Ile-amino)-ethyl-N,N,N-trimethylammoniumacetat
N-2-(BOC-Phe-4-Pia-AHCP-Ile-amino)-ethyl-N,N,N-trimethylammoniumacetat
N-3-(BOC-Phe-2-Pia-AHCP-Ile-amino)-propyl-N,N,N-trimethylammoniumacetat
N-3-(BOC-Phe-3-Pia-AHCP-Ile-amino)-propyl-N,N,N-trimethylammoniumacetat
N-3-(BOC-Phe-4-Pia-AHCP-Ile-amino)-propyl-N,N,N-trimethylammoniumacetat
N-2-(BOC-Phe-Gly-DACP-Leu-amino)-ethyl-N,N,N-trimethylammoniumacetat  N-2-(BOC-Phe-Gly-DACH-Leu-amino)-ethyl-N,N,N-trimethylammoniumacetat
N-2-(BOC-Phe-Gly-DAMH-Leu-amino)-ethyl-N,N,N-trimethylammoniumacetat
N-2-(BOC-Phe-Gly-DAPP-Leu-amino)-ethyl-N,N,N-trimethylammoniumacetat.

Beispiel 3

Eine Lösung von 2,88 g N-2-(H-Ile-amino)-ethyl-N,N,N-trimethylammoniumchlorid-hydrochlorid [F. 89-120°, erhältlich durch Reaktion von BOC-Ile-N-2-dimethylaminoethylamid (F. 100-105°) mit $CH_3I$ zu N-(2-BOC-Ile-aminoethyl)-N,N,N-trimethylammoniumiodid (Öl) und Abspaltung der BOC-Gruppe mit HCl in Dioxan] in 160 ml DMF wird mit 1,01 g N-Methylmorpholin versetzt. Unter Rühren gibt man 5,19 g BOC-Phe-Gly-AHCP-OH, 1,35 g HOBt und eine Lösung von 2,06 g DCCI in 50 ml $CH_2Cl_2$ hinzu, rührt 14 Std. bei 4°, filtriert den ausgeschiedenen Dicyclohexylharnstoff ab und dampft das Filtrat ein. Nach üblicher Aufarbeitung erhält man N-2-(BOC-Phe-Gly-AHCP-Ile-amino)-ethyl-N,N,N-trimethylammonium-acetat ("A"), F. 55-67˚, Rf 0,68.

Analog erhält man:

N-2-(BOC-Phe-βAla-AHCP-Ile-amino)-ethyl-N,N,N-trimethylammoniumacetat

N-2-(BOC-Phe-2-Pya-AHCP-Ile-amino)-ethyl-N,N,N-trimethylammoniumacetat

N-2-(BOC-Phe-3-Pya-AHCP-Ile-amino)-ethyl-N,N,N-trimethylammoniumacetat;Chlorid, F. 140˚; Chlorid-hydrochlorid, F.152-160˚

N-2-(BOC-Phe-4-Pya-AHCP-Ile-amino)-ethyl-N,N,N-trimethylammoniumacetat

N-3-(BOC-Phe-Gly-AHCP-Ile-amino)-propyl-N,N,N-trimethylammoniumacetat, F. 61-72˚, Rf 0,63

N-3-(BOC-Phe-βAla-AHCP-Ile-amino)-propyl-N,N,N-trimethylammoniumacetat

N-3-(BOC-Phe-2-Pya-AHCP-Ile-amino)-propyl-N,N,N-trimethylammoniumacetat

N-3-(BOC-Phe-3-Pya-AHCP-Ile-amino)-propyl-N,N,N-trimethylammoniumacetat

N-3-(BOC-Phe-4-Pya-AHCP-Ile-amino)-propyl-N,N,N-trimethylammoniumacetat

N-4-(BOC-Phe-Gly-AHCP-Ile-amino)-butyl-N,N,N-trimethylammoniumacetat

N-4-(BOC-Phe-βAla-AHCP-Ile-amino)-butyl-N,N,N-trimethylammoniumacetat

N-4-(BOC-Phe-3-Pya-AHCP-Ile-amino)-butyl-N,N,N-trimethylammoniumacetat

N-5-(BOC-Phe-Gly-AHCP-Ile-amino)-pentyl-N,N,N-trimethylammoniumacetat, Rf 0,53

N-5-(BOC-Phe-βAla-AHCP-Ile-amino)-pentyl-N,N,N-trimethylammoniumacetat

N-5-(BOC-Phe-3-Pya-AHCP-Ile-amino)-pentyl-N,N,N-trimethylammoniumacetat.


Beispiel 4

Analog Beispiel 3 erhält man aus BOC-Pro-OH und N-2-(H-Phe-Gly-AHCP-Ile-amino)-ethyl-N,N,N-triethylammoniumchlorid das N-2-(BOC-Pro-Phe-Gly-AHCP-Ile-amino)-ethyl-N,N,N-triethylammoniumchlorid.


Beispiel 5·

Analog Beispiel 3 erhält man aus BOC-Phe-OH und N-2-(H-Gly-AHCP-Ile-amino)-ethyl-N,N,N-trimethylammoniumchlorid "A", F. 55-67˚.

Analog erhält man:

N-2-(BOC-αNal-Gly-AHCP-Ile-amino)-ethyl-N,N,N-trimethylammoniumacetat

N-2-(BOC-βNal-Gly-AHCP-Ile-amino)-ethyl-N,N,N-trimethylammoniumacetat

N-2-(BOC-Trp-Gly-AHCP-Ile-amino)-ethyl-N,N,N-trimethylammoniumacetat

N-2-(BOC-Tyr-Gly-AHCP-Ile-amino)-ethyl-N,N,N-trimethylammoniumacetat

N-2-(Pivaloyl-Phe-Gly-AHCP-Ile-amino)-ethyl-N,N,N-trimethylammoniumacetat

N-2-(3,3-Dimethylbutyryl-Phe-Gly-AHCP-Ile-amino)-ethyl-N,N,N-trimethylammoniumacetat, Rf 0,70

N-2-(3,3-Dimethylbutyryl-Phe-βAla-AHCP-Ile-amino)-ethyl-N,N,N-trimethylammoniumacetat

N-2-(3,3-Dimethylbutyryl-Phe-3-Pya-AHCP-Ile-amino)-ethyl-N,N,N-trimethylammoniumacetat

N-3-(3,3-Dimethylbutyryl-Phe-Gly-AHCP-Ile-amino)-propyl-N,N,N-trimethylammoniumacetat

N-3-(3,3-Dimethylbutyryl-Phe-βAla-AHCP-Ile-amino)-propyl-N,N,N-trimethylammoniumacetat

N-3-(3,3-Dimethylbutyryl-Phe-3-Pya-AHCP-Ile-amino)-propyl-N,N,N-trimethylammoniumacetat

N-2-(MAC-Phe-Gly-AHCP-Ile-amino)-ethyl-N,N,N-trimethylammoniumacetat

N-2-(MAC-Phe-βAla-AHCP-Ile-amino)-ethyl-N,N,N-trimethylammoniumacetat

N-2-(MAC-Phe-3-Pya-AHCP-Ile-amino)-ethyl-N,N,N-trimethylammoniumacetat

N-3-(MAC-Phe-Gly-AHCP-Ile-amino)-propyl-N,N,N-trimethylammoniumacetat

N-3-(MAC-Phe-βAla-AHCP-Ile-amino)-propyl-N,N,N-trimethylammoniumacetat

N-3-(MAC-Phe-3-Pya-AHCP-Ile-amino)-propyl-N,N,N-trimethylammoniumacetat

N-2-(MAC-Phe-Gly-AHCP-Leu-amino)-ethyl-N,N,N-trimethylammoniumacetat

N-2-(MAC-Phe-βAla-AHCP-Leu-amino)-ethyl-N,N,N-trimethylammoniumacetat

N-2-(MAC-Phe-3-Pya-AHCP-Leu-amino)-ethyl-N,N,N-trimethylammoniumacetat

N-2-(3-Dimethylaminopropionyl-Phe-Gly-AHCP-Ile-amino)-ethyl-N,N,N-trimethylammoniumacetat

N-2-(3-Dimethylaminopropionyl-Phe-βAla-AHCP-Ile-amino)-ethyl-N,N,N-trimethylammoniumacetat

N-2-(3-Dimethylaminopropionyl-Phe-3-Pya-AHCP-Ile-amino)-ethyl-N,N,N-trimethylammoniumacetat

N-2-(4-Dimethylaminobutyryl-Phe-Gly-AHCP-Ile-amino)-ethyl-N,N,N-trimethylammoniumacetat

N-2-(5-Dimethylaminopentanoyl-Phe-Gly-AHCP-Ile-amino)-ethyl-N,N,N-trimethylammoniumacetat

N-2-(5-Dimethylaminopentanoyl-Phe-βAla-AHCP-Ile-amino)-ethyl-N,N,N-trimethylammoniumacetat

N-2-(5-Dimethylaminopentanoyl-Phe-3-Pya-AHCP-Ile-amino)-ethyl-N,N,N-trimethylammoniumacetat
N-2-(2-Benzyl-3-dimethylaminopropionyl-Phe-Gly-AHCP-Ile-amino)-ethyl-N,N,N-trimethylammoniumacetat
N-2-(2-Benzyl-3-dimethylaminopropionyl-Phe-βAla-AHCP-Ile-amino)-ethyl-N,N,N-trimethylammoniumacetat
N-2-(2-Benzyl-3-dimethylaminopropionyl-Phe-3-Pya-AHCP-Ile-amino)-ethyl-N,N,N-trimethylammoniumacetat
N-2-(2-Benzyl-5-dimethylaminopentanoyl-Gly-AHCP-Ile-amino)-ethyl-N,N,N-trimethylammoniumacetat
N-2-(2-Benzyl-5-dimethylaminopentanoyl-βAla-AHCP-Ile-amino)-ethyl-N,N,N-trimethylammoniumacetat
N-2-(2-Benzyl-5-dimethylaminopentanoyl-3-Pya-AHCP-Ile-amino)-ethyl-N,N,N-trimethylammoniumacetat
N-2-[2-(2,5-Dioxopyrrolidino)-3-phenylpropionyl-Gly-AHCP-Ile-amino]-ethyl-N,N,N-trimethylammoniumacetat
N-2-[2-(2,5-Dioxopyrrolidino)-3-phenylpropionyl-βAla-AHCP-Ile-amino]-ethyl-N,N,N-trimethylammoniumacetat
N-2-[2-(2,5-Dioxopyrrolidino)-3-phenylpropionyl-3-Pya-AHCP-Ile-amino]-ethyl-N,N,N-trimethylammoniumacetat
N-2-[2-(2,5-Dioxopyrrolidino)-3-phenylpropionyl-Gly-AHCP-Leu-amino]-ethyl-N,N,N-trimethylammoniumacetat
N-2-[2-(2,5-Dioxopyrrolidino)-3-phenylpropionyl-βAla-AHCP-Leu-amino]-ethyl-N,N,N-trimethylammoniumacetat
N-2-[2-(2,5-Dioxopyrrolidino)-3-phenylpropionyl-3-Pya-AHCP-Leu-amino]-ethyl-N,N,N-trimethylammoniumacetat
N-2-[2-(3-Benzyl-2,5-dioxopyrrolidino)-3-phenylpropionyl-Gly-AHCP-Ile-amino]-ethyl-N,N,N-trimethylammoniumacetat
N-3-[2-(2,5-Dioxopyrrolidino)-3-phenylpropionyl-Gly-AHCP-Ile-amino]-propyl-N,N,N-trimethylammoniumacetat, Rf 0,55
N-3-[2-(2,5-Dioxopyrrolidino)-3-phenylpropionyl-βAla-AHCP-Ile-amino]-propyl-N,N,N-trimethylammoniumacetat
N-3-[2-(2,5-Dioxopyrrolidino)-3-phenylpropionyl-3-Pya-AHCP-Ile-amino]-propyl-N,N,N-trimethylammoniumacetat
N-2-(CBZ-Phe-Gly-AHCP-Ile-amino)-ethyl-N,N,N-trimethylammoniumacetat.

Beispiel 6

Analog Beispiel 3 erhält man aus BOC-Phe-3-Pya-OH und N-2-(H-AHCP-Ile-amino)-ethyl-N,N,N-trimethylammoniumchlorid das N-2-(BOC-Phe-3-Pya-AHCP-Ile-amino)-ethyl-N,N,N-trimethylammoniumchlorid, F. 225-230°.

Analog erhält man:
N-2-(BOC-Phe-Gly-AHCP-Leu-amino)-ethyl-N,N,N-trimethylammoniumchlorid, F. 141-142°
N-2-(BOC-Phe-βAla-AHCP-Leu-amino)-ethyl-N,N,N-trimethylammoniumchlorid
N-2-(BOC-Phe-2-Pya-AHCP-Leu-amino)-ethyl-N,N,N-trimethylammoniumchlorid
N-2-(BOC-Phe-3-Pya-AHCP-Leu-amino)-ethyl-N,N,N-trimethylammoniumchlorid
N-2-(BOC-Phe-4-Pya-AHCP-Leu-amino)-ethyl-N,N,N-trimethylammoniumchlorid
N-3-(BOC-Phe-Gly-AHCP-Leu-amino)-propyl-N,N,N-trimethylammoniumchlorid
N-3-(BOC-Phe-βAla-AHCP-Leu-amino)-propyl-N,N,N-trimethylammoniumchlorid
N-3-(BOC-Phe-2-Pya-AHCP-Leu-amino)-propyl-N,N,N-trimethylammoniumchlorid
N-3-(BOC-Phe-3-Pya-AHCP-Leu-amino)-propyl-N,N,N-trimethylammoniumchlorid
N-3-(BOC-Phe-4-Pya-AHCP-Leu-amino)-propyl-N,N,N-trimethylammoniumchlorid
N-2-(BOC-Phe-Gly-AHCP-Nle-amino)-ethyl-N,N,N-trimethylammoniumchlorid
N-2-(BOC-Phe-βAla-AHCP-Nle-amino)-ethyl-N,N,N-trimethylammoniumchlorid
N-2-(BOC-Phe-3-Pya-AHCP-Nle-amino)-ethyl-N,N,N-trimethylammoniumchlorid
N-2-(BOC-Phe-Gly-AHCP-Phe-amino)-ethyl-N,N,N-trimethylammoniumchlorid
N-2-(BOC-Phe-βAla-AHCP-Phe-amino)-ethyl-N,N,N-trimethylammoniumchlorid
N-2-(BOC-Phe-3-Pya-AHCP-Phe-amino)-ethyl-N,N,N-trimethylammoniumchlorid
N-2-(BOC-Phe-Gly-AHCP-Val-amino)-ethyl-N,N,N-trimethylammoniumchlorid
N-2-(BOC-Phe-βAla-AHCP-Val-amino)-ethyl-N,N,N-trimethylammoniumchlorid
N-2-(BOC-Phe-3-Pya-AHCP-Val-amino)-ethyl-N,N,N-trimethylammoniumchlorid
N-2-(BOC-Phe-Gly-Sta-Ile-amino)-ethyl-N,N,N-trimethylammoniumchlorid
N-2-(BOC-Phe-Gly-AHCH-Ile-amino)-ethyl-N,N,N-trimethylammoniumchlorid
N-2-(BOC-Phe-Gly-AHPP-Ile-amino)-ethyl-N,N,N-trimethylammoniumchlorid
N-2-(2-Benzyl-4-oxo-5,5-dimethyl-hexanoyl-Gly-AHCP-Ile-amino)-ethyl-N,N,N-trimethylammoniumacetat, Rf 0,68

N-2-(2-Isopropyl-4S-hydroxy-5S-BOC-Phe-βAla-amino-6-cyclohexyl-hexanoyl-amino)-ethyl-N,N,N-trimethylammoniumchlorid, F. 129-130˚

N-2-(N-Benzoyl-N-benzyl-Gly-AHCP-Ile-amino)-ethyl-pyridiniumbromid

N-2-[2-(2,5-Dioxopyrrolidino)-3-(p-hydroxyphenyl)-propionyl-Gly-AHCP-Ile-amino]-ethyl-N,N,N-trimethylammonium-methansulfonat

N-2-[2-(2,5-Dioxopyrrolidino)-3-(3-indolyl)-propionyl-Gly-AHCP-Ile-amino]-ethyl-N,N,N-tripropylammoniumchlorid

N-2-[2-(2,5-Dioxopyrrolidino)-3-(1-naphthyl)-propionyl-Gly-AHCP-Ile-amino]-ethyl-N,N,N-tributylammoniumchlorid

N-2-[2-(2,5-Dioxopyrrolidino)-3-(2-naphthyl)-propionyl-Gly-AHCP-Ile-amino]-ethyl-N-2-phenylethyl-morpholiniumchlorid

N-2-[(3-Benzyl-2,5-dioxopyrrolidino)-acetyl-AHCP-Ile-amino]-ethyl-N,N,N-trimethylammoniumacetat

N-2-[2-(3-Benzyl-2,5-dioxopyrrolidino)-propionyl-AHCP-Ile-amino]-ethyl-N,N,N-trimethylammoniumacetat

N-2-[2-(3-Benzyl-2,5-dioxopyrrolidino)-3-(4-imidazolyl)-propionyl-ACHP-Ile-amino]-ethyl-N,N,N-trimethylammoniumacetat

N-2-(2-Benzyl-4-dimethylamino-4-oxobutyryl-Gly-AHCP-Ile-amino)-ethyl-N,N,N-trimethylammoniumacetat

N-2-(2-Benzyl-4-dimethylamino-4-oxobutyryl-βAla-AHCP-Ile-amino)-ethyl-N,N,N-trimethylammoniumacetat

N-2-(2-Benzyl-4-dimethylamino-4-oxobutyryl-3-Pya-AHCP-Ile-amino)-ethyl-N,N,N-trimethylammoniumacetat

N-2-(2-Benzyl-4-(3-dimethylaminopropyl)-4-oxobutyryl-Gly-AHCP-Ile-amino)-ethyl-N,N,N-trimethylammoniumacetat

N-2-(2-Benzyl-4-(3-dimethylaminopropyl)-4-oxobutyryl-βAla-AHCP-Ile-amino)-ethyl-N,N,N-trimethylammoniumacetat

N-2-(2-Benzyl-4-(3-dimethylaminopropyl)-4-oxobutyryl-3-Pya-AHCP-Ile-amino)-ethyl-N,N,N-trimethylammoniumacetat

N-2-(2-Benzyl-4-oxo-5,5-dimethyl-hexanoyl-Gly-AHCP-Ile-amino)-ethyl-N,N,N-trimethylammoniumacetat

N-2-(2-Benzyl-4-oxo-5,5-dimethyl-hexanoyl-βAla-AHCP-Ile-amino)-ethyl-N,N,N-trimethylammoniumacetat

N-2-(2-Benzyl-4-oxo-5,5-dimethyl-hexanoyl-3-Pya-AHCP-Ile-amino)-ethyl-N,N,N-trimethylammoniumacetat

N-2-(2-Dimethylamino-3-phenyl-propionyl-Gly-AHCP-Ile-amino)-ethyl-N,N,N-trimethylammoniumacetat

N-2-(2-Dimethylamino-3-phenyl-propionyl-βAla-AHCP-Ile-amino)-ethyl-N,N,N-trimethylammoniumacetat

N-2-(2-Dimethylamino-3-phenyl-propionyl-3-Pya-AHCP-Ile-amino)-ethyl-N,N,N-trimethylammoniumacetat.


Beispiel 7

Analog Beispiel 3 erhält man aus BOC-Phe-Gly-AHCP-Ile-OH und N-2-Aminoethyl-N,N,N-trimethylammoniumchlorid "A", F. 55-67˚.

Analog erhält man:

N-4-(BOC-Phe-Gly-AHCP-Ile-amino)-butyl-N,N,N-trimethylammoniumacetat

N-5-(BOC-Phe-Gly-AHCP-Ile-amino)-pentyl-N,N,N-trimethylammoniumacetat

N-6-(BOC-Phe-Gly-AHCP-Ile-amino)-hexyl-N,N,N-trimethylammoniumacetat

N-7-(BOC-Phe-Gly-AHCP-Ile-amino)-heptyl-N,N,N-trimethylammoniumacetat

N-8-(BOC-Phe-Gly-AHCP-Ile-amino)-octyl-N,N,N-trimethylammoniumacetat

N-9-(BOC-Phe-Gly-AHCP-Ile-amino)-nonyl-N,N,N-trimethylammoniumacetat

N-10-(BOC-Phe-Gly-AHCP-Ile-amino)-decyl-N,N,N-trimethylammoniumacetat

N-p-(BOC-Phe-Gly-AHCP-Ile-aminomethyl)-benzyl-N,N,N-trimethylammoniumacetat

N-2-(BOC-Phe-Gly-AHCP-Ile-amino)-ethyl-N,N-dimethyl-N-hexyl-ammoniumacetat

N-2-(BOC-Phe-Gly-AHCP-Ile-amino)-ethyl-N,N-dimethyl-N-octyl-ammoniumacetat

N-2-(BOC-Phe-βAla-AHCP-Ile-amino)-ethyl-N,N-dimethyl-N-octyl-ammoniumacetat

N-2-(BOC-Phe-3-Pya-AHCP-Ile-amino)-ethyl-N,N-dimethyl-N-octyl-ammoniumacetat

N-3-(BOC-Phe-Gly-AHCP-Ile-amino)-propyl-N,N-dimethyl-N-octyl-ammoniumacetat

N-3-(BOC-Phe-βAla-AHCP-Ile-amino)-propyl-N,N-dimethyl-N-octyl-ammoniumacetat

N-3-(BOC-Phe-3-Pya-AHCP-Ile-amino)-propyl-N,N-dimethyl-N-octyl-ammoniumacetat

N-5-(BOC-Phe-Gly-AHCP-Ile-amino)-pentyl-N,N-dimethyl-N-octyl-ammoniumacetat

N-5-(BOC-Phe-βAla-AHCP-Ile-amino)-pentyl-N,N-dimethyl-N-octyl-ammoniumacetat

N-5-(BOC-Phe-3-Pya-AHCP-Ile-amino)-pentyl-N,N-dimethyl-N-octyl-ammoniumacetat

N-2-(BOC-Phe-Gly-AHCP-Ile-amino)-ethyl-N,N-dimethyl-N-decyl-ammoniumacetat

N-2-(BOC-Phe-Gly-AHCP-Ile-amino)-ethyl-N-methyl-pyrrolidiniumacetat

N-3-(BOC-Phe-Gly-AHCP-Ile-amino)-propyl-N-methyl-pyrrolidiniumacetat
N-2-(BOC-Phe-Gly-AHCP-Ile-amino)-ethyl-N-methyl-piperidiniumacetat
N-3-(BOC-Phe-Gly-AHCP-Ile-amino)-propyl-N-methyl-piperidiniumacetat
N-2-(BOC-Phe-Gly-AHCP-Ile-amino)-ethyl-N-methyl-morpholiniumacetat
N-3-(BOC-Phe-Gly-AHCP-Ile-amino)-propyl-N-methyl-morpholiniumacetat
N-Benzyl-4-(BOC-Phe-Gly-AHCP-Ile-amino)-N-methyl-piperidiniumacetat
3-(BOC-Phe-Gly-AHCP-Ile-aminomethyl)-N-methyl-pyridiniumchlorid, F. 138°
N-2-(BOC-Phe-Gly-AHCP-Ile-amino)-ethyl-pyridiniumacetat.

## Beispiel 8

Analog Beispiel 3 erhält man aus BOC-Phe-Gly-AHCP-Ala-OH und N-2-(H-Leu-amino)-ethyl-N,N,N-trimethylammoniumchlorid das N-2-(BOC-Phe-Gly-AHCP-Ala-Leu-amino)-ethyl-N,N,N-trimethylammoniumchlorid.

## Beispiel 9

Zu einer Suspension von 1 g BOC-Phe-Gly-AHCP-Ile-N-(2-dimethylaminoethyl)-amid in 12 ml Acetonitril tropft man bei 0° unter Rühren eine Lösung von 0,4 g $CH_3I$ in 2 ml Acetonitril, rührt noch 4 Std. bei 20°, dampft ein, wäscht das Rohprodukt mehrfach mit Ether, arbeitet wie üblich auf und erhält "A", F. 55-67°.

Das gleiche Produkt wird analog aus äquimolekularen Mengen BOC-Phe-Gly-AHCP-Ile-N-(2-chlorethyl)-amid und Trimethylamin erhalten.

Analog erhält man aus IPOC-Phe-Gly-AHCP-Ile-(3-brompropylester) und N-Methyl-morpholin das N-3-(IPOC-Phe-Gly-AHCP-Ile-oxy)-propyl-N-methyl-morpholiniumbromid.

## Beispiel 10

Eine Lösung von 1 g N-2-(BOC-Phe-Gly-AHCP-Ile-amino)-ethyl-N,N,N-trimethylammoniumacetat in 20 ml 4 n HCl in Dioxan wird 30 Min. bei 20° gerührt und dann eingedampft. Man erhält N-2-(H-Phe-Gly-AHCP-Ile-amino)-ethyl-N,N,N-trimethylammoniumchlorid-hydrochlorid.

## Beispiel 11

Analog Beispiel 2 erhält man aus N-2-(CBZ-Phe-Gly-AHCP-Ile-amino)-ethyl-N,N,N-trimethylammoniumacetat durch Hydrogenolyse das N-2-(H-Phe-Gly-AHCP-Ile-amino)-ethyl-N,N,N-trimethylammoniumacetat.

## Beispiel 12

Eine Lösung von 751 mg N-2-[N-(3-Oxo-4S-BOC-Phe-Gly-amino-5-cyclohexylpentanoyl)-Ile-amino]-ethyl-N,N,N-trimethylammoniumchlorid und 1,43 g $Na_2CO_3$ . 10 $H_2O$ in 5 ml Methanol und 5 ml Wasser wird mit 70 mg Hydroxylaminhydrochlorid versetzt und 14 Std. bei 20° gerührt. Das ausgefallene Oxim wird abfiltriert, getrocknet, in 10 ml Methanol gelöst und an 0,5 g Raney-Ni bei 20° und 5 bar hydriert. Man filtriert den Katalysator ab, dampft das Filtrat ein, trennt das erhaltene Gemisch an Kieselgel und erhält N-2-[N-(3S-Amino-4S-BOC-Phe-Gly-amino-5-cyclohexylpentanoyl)-Ile-amino]-ethyl-N,N,N-trimethylammoniumchlorid ["N-2-(BOC-Phe-Gly-DACP-Ile-amino)-ethyl-N,N,N-trimethylammoniumchlorid"]; daneben erhält man das 3R-Amino-Epimere.

Analog erhält man aus den entsprechenden Oxoverbindungen:
N-2-(BOC-Phe-Gly-DACH-Ile-amino)-ethyl-N,N,N-trimethylammoniumchlorid,
N-2-(BOC-Phe-Gly-DAMH-Ile-amino)-ethyl-N,N,N-trimethylammoniumchlorid,
N-2-(BOC-Phe-Gly-DAPP-Ile-amino)-ethyl-N,N,N-trimethylammoniumchlorid.

Beispiel 13

Analog Beispiel 2, jedoch in Essigsäure statt Ethanol, erhält man durch Hydrogenolyse von BOC-Phe-imi-BOM-His-AHCP-Ile-AMPA-methoiodid das BOC-Phe-His-AHCP-Ile-AMPA-methoacetat, F. 121°.

Analog erhält man durch Hydrogenolyse der Methoiodide der entsprechenden imi-BOM-Derivate die Methoacetate folgender Verbindungen:

6-Aminohexanoyl-Phe-His-AHCP-Ile-AMPA
6-Aminohexanoyl-Phe-His-AHCP-Leu-AMPA
6-Dimethylaminohexanoyl-Phe-His-AHCP-Ile-AMPA
6-Dimethylaminohexanoyl-Phe-His-AHCP-Leu-AMPA
6-Aminohexanoyl-Phe-His-AHCP-Ile-(N-2-pyridylmethylamid)
6-Aminohexanoyl-Phe-His-AHCP-Leu-(N-2-pyridylmethylamid)
6-Aminohexanoyl-Phe-His-AHCP-Ile-(N-3-pyridylmethylamid)
6-Aminohexanoyl-Phe-His-AHCP-Leu-(N-3-pyridylmethylamid)
6-Aminohexanoyl-Phe-His-AHCP-Ile-(N-4-pyridylmethylamid)
6-Aminohexanoyl-Phe-His-AHCP-Leu-(N-4-pyridylmethylamid)
6-Aminohexanoyl-Phe-His-AHCP-Ile-(N-2-hydroxy-4,6-dimethyl-3-pyridylmethylamid)
6-Aminohexanoyl-Phe-His-AHCP-Leu-(N-2-hydroxy-4,6-dimethyl-3-pyridylmethylamid).


Beispiel 14

Analog Beispiel 3 erhält man aus 4-Dimethylaminobuttersäure und H-Phe-Gly-AHCP-Ile-AMPA-methoiodid das 4-Dimethylaminobutyryl-Phe-Gly-AHCP-Ile-AMPA-methoiodid.

Analog erhält man:
4-Dimethylaminobutyryl-Phe-Gly-AHCP-Ile-ADPA-methoiodid
4-Dimethylaminobutyryl-Phe-Gly-AHCP-Leu-AMPA-methoiodid
4-Dimethylaminobutyryl-Phe-Gly-AHCP-Leu-ADPA-methoiodid
4-Dimethylaminobutyryl-Phe-βAla-AHCP-Ile-AMPA-methoiodid
4-Dimethylaminobutyryl-Phe-βAla-AHCP-Ile-ADPA-methoiodid
4-Dimethylaminobutyryl-Phe-βAla-AHCP-Leu-AMPA-methoiodid
4-Dimethylaminobutyryl-Phe-βAla-AHCP-Leu-ADPA-methoiodid
6-Dimethylaminohexanoyl-Phe-Gly-AHCP-Ile-AMPA-methoiodid
6-Dimethylaminohexanoyl-Phe-Gly-AHCP-Leu-AMPA-methoiodid
6-Dimethylaminohexanoyl-Phe-βAla-AHCP-Ile-AMPA-methoiodid
6-Dimethylaminohexanoyl-Phe-βAla-AHCP-Leu-AMPA-methoiodid.


Beispiel 15

Analog Beispiel 9 erhält man aus 6-BOC-aminohexanoyl-Phe-Gly-AHCP-Ile-(N-3-pyridylmethylamid) und CH₃I das entsprechende Methoiodid, Zers. bei 96°.

Analog erhält man
BOC-Phe-Gly-AHCP-Ile-AMPA-methochlorid, F. 150°
4-BOC-aminobutyryl-Phe-Gly-AHCP-Ile-AMPA-methoiodid
4-BOC-aminobutyryl-Phe-Gly-AHCP-Ile-ADPA-methoiodid
4-BOC-aminobutyryl-Phe-Gly-AHCP-Leu-AMPA-methoiodid
4-BOC-aminobutyryl-Phe-Gly-AHCP-Leu-ADPA-methoiodid
6-BOC-aminohexanoyl-Phe-Gly-AHCP-Ile-AMPA-methoiodid, F. 111°
6-BOC-aminohexanoyl-Phe-Gly-AHCP-Ile-ADPA-methoiodid
6-BOC-aminohexanoyl-Phe-Gly-AHCP-Ile-(N-2-pyridylmethylamid)-methoiodid
6-BOC-aminohexanoyl-Phe-Gly-AHCP-Ile-(N-4-pyridylmethylamid)-methoiodid
6-BOC-aminohexanoyl-Phe-Gly-AHCP-Ile-(N-2-hydroxy-4,6-dimethyl-3-pyridylmethylamid)-methoiodid
6-BOC-aminohexanoyl-Phe-Gly-AHCP-Leu-AMPA-methoiodid
6-BOC-aminohexanoyl-Phe-Gly-AHCP-Leu-ADPA-methoiodid
6-BOC-aminohexanoyl-Phe-Gly-AHCP-Leu-(N-2-pyridylmethylamid)-methoiodid
6-BOC-aminohexanoyl-Phe-Gly-AHCP-Leu-(N-3-pyridylmethylamid)-methoiodid
6-BOC-aminohexanoyl-Phe-Gly-AHCP-Leu-(N-4-pyridylmethylamid)-methoiodid

6-BOC-aminohexanoyl-Phe-Gly-AHCP-Leu-(N-2-hydroxy-4,6-dimethyl-3-pyridylmethylamid)-methoiodid
6-BOC-aminohexanoyl-Phe-βAla-AHCP-Ile-AMPA-methoiodid
6-BOC-aminohexanoyl-Phe-βAla-AHCP-Ile-ADPA-methoiodid
6-BOC-aminohexanoyl-Phe-βAla-AHCP-Ile-(N-2-pyridylmethylamid)-methoiodid
6-BOC-aminohexanoyl-Phe-βAla-AHCP-Ile-(N-3-pyridylmethylamid)-methoiodid
6-BOC-aminohexanoyl-Phe-βAla-AHCP-Ile-(N-4-pyridylmethylamid)-methoiodid
6-BOC-aminohexanoyl-Phe-βAla-AHCP-Ile-(N-2-hydroxy-4,6-dimethyl-3-pyridylmethylamid)-methoiodid
6-BOC-aminohexanoyl-Phe-βAla-AHCP-Leu-AMPA-methoiodid
6-BOC-aminohexanoyl-Phe-βAla-AHCP-Leu-ADPA-methoiodid
6-BOC-aminohexanoyl-Phe-βAla-AHCP-Leu-(N-2-pyridylmethylamid)-methoiodid
6-BOC-aminohexanoyl-Phe-βAla-AHCP-Leu-(N-3-pyridylmethylamid)-methoiodid
6-BOC-aminohexanoyl-Phe-βAla-AHCP-Leu-(N-4-pyridylmethylamid)-methoiodid
6-BOC-aminohexanoyl-Phe-βAla-AHCP-Leu-(N-2-hydroxy-4,6-dimethyl-3-pyridylmethylamid)-methoiodid
8-BOC-aminooctanoyl-Phe-Gly-AHCP-Ile-AMPA-methoiodid
8-BOC-aminooctanoyl-Phe-Gly-AHCP-Ile-ADPA-methoiodid
8-BOC-aminooctanoyl-Phe-Gly-AHCP-Leu-AMPA-methoiodid
8-BOC-aminooctanoyl-Phe-Gly-AHCP-Leu-ADPA-methoiodid.

Beispiel 16

Durch einwöchiges Stehen einer ethanolischen Lösung von 2-tert.-Butylamino-3-phenylpropionyl-Gly-AHCP-Ile-AMPA mit überschüssigem CH₃I erhält man das entsprechende (Mono)-methiodid, F. 180°.

Beispiel 17

Eine Lösung von 1 g (2-Benzyl-4-oxo-5,5-dimethyl-hexanoyl)-imi-tert.-butoxy-methyl-His-AHCP-Ile-AMPA-methoiodid[erhältlich durch Kondensation von 2-Benzyl-4-oxo-5,5-dimethylhexansäure mit H-(imi-tert.-Butoxymethyl-His)-AHCP-Ile-AMPA und anschließende Reaktion mit CH₃I] in 20 ml 4 n HCl in Dioxan wird 30 Min. bei 20° gerührt und dann eingedampft. Man erhält 2-Benzyl-4-oxo-5,5-dimethyl-hexanoyl-His-AHCP-Ile-AMPA-methochlorid-hydrochlorid, Zers. bei 102°.
Analog erhält man durch Spaltung der Methoiodide der entsprechenden BOC-amino-derivate:
4-Aminobutyryl-Phe-Gly-AHCP-Ile-AMPA-methochlorid
4-Aminobutyryl-Phe-Gly-AHCP-Ile-ADPA-methochlorid
4-Aminobutyryl-Phe-Gly-AHCP-Leu-AMPA-methochlorid
4-Aminobutyryl-Phe-Gly-AHCP-Leu-ADPA-methochlorid
6-Aminohexanoyl-Phe-Gly-AHCP-Ile-AMPA-methochlorid, Dihydrochlorid, Zers. bei 102°
6-Aminohexanoyl-Phe-Gly-AHCP-Ile-ADPA-methochlorid
6-Aminohexanoyl-Phe-Gly-AHCP-Ile-(N-2-pyridylmethylamid)-methochlorid
6-Aminohexanoyl-Phe-Gly-AHCP-Ile-(N-3-pyridylmethylamid)-methochlorid, Dihydrochlorid, Zers. bei 105°
6-Aminohexanoyl-Phe-Gly-AHCP-Ile-(N-4-pyridylmethylamid)-methochlorid
6-Aminohexanoyl-Phe-Gly-AHCP-Ile-(N-2-hydroxy-4,6-dimethyl-3-pyridylmethylamid)-methochlorid
6-Aminohexanoyl-Phe-Gly-AHCP-Leu-AMPA-methochlorid
6-Aminohexanoyl-Phe-Gly-AHCP-Leu-ADPA-methochlorid
6-Aminohexanoyl-Phe-Gly-AHCP-Leu-(N-2-pyridylmethylamid)-methochlorid
6-Aminohexanoyl-Phe-Gly-AHCP-Leu-(N-3-pyridylmethylamid)-methochlorid
6-Aminohexanoyl-Phe-Gly-AHCP-Leu-(N-4-pyridylmethylamid)-methochlorid
6-Aminohexanoyl-Phe-Gly-AHCP-Leu-(N-2-hydroxy-4,6-dimethyl-3-pyridylmethylamid)-methochlorid
8-Aminooctanoyl-Phe-Gly-AHCP-Ile-AMPA-methochlorid
8-Aminooctanoyl-Phe-Gly-AHCP-Ile-ADPA-methochlorid
8-Aminooctanoyl-Phe-Gly-AHCP-Leu-AMPA-methochlorid
8-Aminooctanoyl-Phe-Gly-AHCP-Leu-ADPA-methochlorid.
Die nachfolgenden Beispiele betreffen pharmazeutische Zubereitungen.

Beispiel A: Injektionsgläser

Eine Lösung von 100 g N-2-(BOC-Phe-Gly-AHCP-Ile-amino)-ethyl-N,N,N-trimethylammoniumacetat und 5 g Dinatriumhydrogenphosphat in 4 l zweifach destilliertem Wasser wird mit 2 n Salzsäure auf pH 6,5 eingestellt, steril filtriert und in Injektionsgläser abgefüllt. Man lyophilisiert unter sterilen Bedingungen und verschließt steril. Jedes Injektionsglas enthält 500 mg Wirkstoff.

Beispiel B: Suppositorien

Man schmilzt ein Gemisch von 50 g N-2-(BOC-Phe-Gly-ACHP-Ile-amino)-ethyl-N,N,N-trimethylammoniumchlorid mit 10 g Sojalecithin und 140 g Kakaobutter, gießt in Formen und läßt erkalten. Jedes Suppositorium enthält 250 mg Wirkstoff.

Beispiel C: Tabletten

Ein Gemisch von 1 kg 6-Aminohexanoyl-Phe-Gly-AHCP-Ile-AMPA-methochlorid-dihydrochlorid, 4 kg Lactose, 1,2 kg Maisstärke, 200 g Talk und 100 g Magnesiumstearat wird in üblicher Weise zu Tabletten verpreßt, derart daß jede Tablette 100 mg Wirkstoff enthält.

Beispiel D: Dragees

Analog Beispiel A werden Tabletten gepreßt, die anschließend in üblicher Weise mit einem Überzug aus Saccharose, Maisstärke, Talk, Tragant und Farbstoff überzogen werden.

Beispiel E: Kapseln

500 g 6-Aminohexanoyl-Phe-Gly-AHCP-Ile-(N-3-pyridylmethylamid)-methochlorid-dihydrochlorid werden in üblicher Weise in Hartgelatinekapseln gefüllt, so daß jede Kapsel 500 mg Wirkstoff enthält.

## Ansprüche

1. Aminosäurederivate der Formel I

$$R^1\text{-Z-M-}NR^2\text{-}CHR^3\text{-}CR^4\text{-}(CHR^5)_n\text{-CO-E-Q-Y } An^{\ominus} \qquad I$$

worin

$R^1$    $R^6$, $R^6$-O-$C_mH_{2m}$-CO-, $R^6$-$C_mH_{2m}$-O-CO-, $R^6$-$C_mH_{2m}$-CO-, $R^6$-$SO_2$-, ($R^6$-$C_mH_{2m}$-(T)$_x$-(G)$_y$-$C_rH_{2r}$)-L($R^7$-$C_pH_{2p}$)-$C_tH_{2t}$-CO-, $R^6$-($NHCH_2CH_2$)$_m$-$NHCH_2$CO-, oder 9-Fluorenyl-$C_mH_{2m}$-O-CO-,

Z    0 bis 3 peptidartig miteinander verbundene Aminosäurereste ausgewählt aus der Gruppe bestehend aus Abu, Ada, Ala, βAla, Arg, Asn, Asp, Bia, Cal, Dab, Gln, Glu, Gly, His, N(im)-A-His, Ile, Leu, tert.-Leu, Lys, Met, αNal, βNal, Nbg, Nle, Orn, Phe, Pia, Pro, Pya, Ser, Thr, Tic, Trp, Tyr und Val,

M    βAla, Gly, Pia, Pya, oder, falls im Rest Y anstelle eines N-Atoms einer Het-ylengruppe eine $R^8N^{\oplus}$-Gruppe steht, auch His,

E    0 bis 2 peptidartig miteinander verbundene Aminosäurereste ausgewählt aus der Gruppe bestehend aus Abu, Ala, Cal, His, Ile, Leu, Met, Nle, Nva, Phe, Trp, Tyr und Val,

Q    O oder NH,

Y    eine Alkylenkette, worin auch eine $CH_2$-Gruppe durch eine Ar-ylengruppe oder Hetylengruppe ersetzt sein kann, mit insgesamt 1-20 C-Atomen, welche durch eine $R^8R^9R^{10}N^{\oplus}$-Gruppe oder eine unsubstituierte oder eine ein- oder mehrfach durch A, AO und/oder Hal substituierte Pyridiniumgruppe substituiert ist, und/oder in welcher an Stelle eines N-Atoms einer Hetylengruppe eine $R^8N^{\oplus}$-Gruppe steht,

$An^{\ominus}$    ein Anion, das auch fehlen kann, wenn stattdessen eine in der Verbindung der Formel I enthaltene Carboxygruppe in Form eines Carboxylations vorliegt,

$R^3$, $R^6$ und $R^7$    jeweils H, A, Ar, Ar-alkyl, Het, Het-alkyl, unsubstituiertes oder ein- oder mehrfach durch A, AO und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen,

$R^2$ und $R^5$    jeweils H oder A,

20

EP 0 327 877 A2

R⁴ (H, OH), (H, NH₂) oder = O,

R⁸, R⁹ und R¹⁰ jeweils Alkyl mit 1-18 C-Atomen oder Aralkyl, zwei der Reste R⁸, R⁹ und R¹⁰ zusammen auch eine Alkylengruppe mit 2-8 C-Atomen, die durch ein O-Atom oder durch eine NR¹¹-Gruppe unterbrochen sein kann,

R¹¹ H, A, Ar oder Ar-alkyl,

L CH oder N,

T O, S, NH oder NA,

G CO, S, SO oder SO₂,

n 1 oder 2,

m, p, r und t jeweils 0, 1, 2, 3, 4 oder 5,

x und y jeweils 0 oder 1,

Ar unsubstituiertes oder ein- oder mehrfach durch A, AO, Hal, CF₃, OH, H₂NSO₂ und/oder NH₂ substituiertes Phenyl oder unsubstituiertes Naphthyl,

Het einen gesättigten oder ungesättigten 5-oder 6-gliedrigen heterocyclischen Rest mit 1-4 N-, O- und/oder S-Atomen, der mit einem Benzolring kondensiert und/oder ein-oder mehrfach durch A, AO, Hal, CF₃, HO, O₂N, Carbonylsauerstoff, H₂N, HAN, A₂N, AcNH, AS, ASO, ASO₂, HOOC, AOOC, CN, H₂NCO, H₂NSO₂, ASO₂NH, Ar, Ar-alkyl, Ar-alkenyl, Hydroxyalkyl und/oder Aminoalkyl mit jeweils 1-8 C-Atomen substituiert sein kann und/oder dessen N- und/oder S-Heteroatome auch oxydiert sein können,

Hal F, Cl, Br oder J,

Ac A-CO-, Ar-CO- oder A-NH-CO-,

-alkyl- eine Alkylengruppe mit 1-4 C-Atomen und

A Alkyl mit 1-8 C-Atomen bedeuten,

worin ferner an Stelle einer oder mehrerer -NH-CO-Gruppen auch eine oder mehrere NA-CO-Gruppen stehen können,

sowie deren Salze.

2.
   a) Salze des N-2-(BOC-Phe-Gly-AHCP-Ile-amino)-ethyl-N,N,N-trimethylammoniumhydroxids.
   b) Salze des N-3-(BOC-Phe-Gly-AHCP-Ile-amino)-propyl-N,N,N-trimethylammoniumhydroxids.
   c) Salze des N-2-(BOC-Phe-3-Pya-AHCP-Ile-amino)-ethyl-N,N,N-trimethylammoniumhydroxids.
   d) Salze des N-Methyl-3-(6-aminohexanoyl-Phe-Gly-AHCP-Ile-aminomethyl)-pyridiniumhydroxids.
   e) Salze des 1,2- (oder 2,3-) Dimethyl-4-amino-5-(6-aminohexanoyl-Phe-Gly-AHCP-Ile-aminomethyl)-pyrimidiniumhydroxids.

3. Verfahren zur Herstellung eines Aminosäurederivats der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt oder daß man eine Carbonsäure der Formel II

R¹-G¹-OH    II

worin G¹

(a) Z¹,

(b) Z,

(c) Z-M,

(d) Z-M-W,

(e) Z-M-W-E¹,

(f) Z-M-W-E und

W -NR²-CHR³-CR⁴-(CHR⁵)ₙ-CO- bedeuten

mit einer Aminoverbindung der Formel III

H-G²    III

worin

G²

(a) -Z²-M-W-E-Q-Y,

(b) -M-W-E-Q-Y,

(c) -W-E-Q-Y,

(d) -E-Q-Y,

(e) -E²-Q-Y,

(d) NH-Y,

E¹ + E² zusammen E und

Z¹ + Z² zusammen Z bedeuten

umsetzt,

21

oder daß man ein sonst der Formel I entsprechendes tertiäres Amin mit einem quaternisierenden Mittel behandelt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder zur Herstellung einer Verbindung der Formel I, $R^4$ = (H, OH) oder (H, $NH_2$) ein Aminoketosäurederivat der Formel I, $R^4$ = O, reduziert oder reduktiv aminiert und/oder eine Verbindung der Formel I durch Behandeln mit einer Säure in eines ihrer Salze überführt und/oder in einer Verbindung der Formel I ein Anion $An^\ominus$ gegen ein anderes Anion $An^\ominus$ austauscht.

4. Verfahren zur Herstellung pharmazeutischer Zubereitungen, dadurch gekennzeichnet, daß man eine Verbindung der Formel I und/oder eines ihres physiologisch unbedenklichen Salzes zusammen mit mindestens einem festen, flüssigen oder halbflüssigen Träger-oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I oder von deren physiologisch unbedenklichen Salzen zur Herstellung eines Arzneimittels.

7. Verwendung von Verbindungen der Formel I oder von deren physiologisch unbedenklichen Salzen bei der Bekämpfung der reninabhängigen Hypertension oder des Hyperaldosteronismus.